(19) Europäisches Patentamt
European Patent Office
Office européen des brevets

(11) **EP 4 424 231 A1**

(12) **EUROPEAN PATENT APPLICATION**

(43) Date of publication:
04.09.2024 Bulletin 2024/36

(51) International Patent Classification (IPC):
*A61B 5/00* (2006.01)  *A61B 5/024* (2006.01)
*A61B 5/08* (2006.01)  *A61B 5/11* (2006.01)
*A61B 5/113* (2006.01)

(21) Application number: 24160886.8

(22) Date of filing: 01.03.2024

(52) Cooperative Patent Classification (CPC):
A61B 5/7267; A61B 5/0077; A61B 5/024;
A61B 5/0816; A61B 5/1118; A61B 5/1128;
A61B 5/113; A61B 5/4812

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 03.03.2023 GB 202303148
26.02.2024 GB 202402664

(71) Applicant: Oxehealth Limited
The Oxford Science Park
Oxford
OX4 4GA (GB)

(72) Inventors:
• CARTER, Jonathan Frederick
  Oxford, OX4 4GA (GB)
• TARASSENKO, Lionel
  Oxford, OX4 4GA (GB)
• JORGE, Joao Goncalo Malveiro
  Oxford, OX4 4GA (GB)
• GIBSON, Oliver John
  Oxford, OX4 4GA (GB)

(74) Representative: J A Kemp LLP
80 Turnmill Street
London EC1M 5QU (GB)

(54) **MACHINE LEARNING CLASSIFICATION OF SLEEP STATE**

(57) A method comprising determining a sleep state of a test subject using input data from the test subject. The input data comprise: at least one measure of test subject movement; and at least one cardiorespiratory feature of the test subject and are derived from video images of the test subject. The at least one cardiorespiratory feature of the test subject is derived using a feature extractor trained using reference cardiorespiratory signals for each of a plurality of reference subjects derived from time-resolved measurements from a plurality of sensors worn by the reference subjects, the feature extractor comprising a neural network, and the reference cardiorespiratory signals. Also provided a method of training a machine-learning algorithm to determine a sleep state of a subject using training data in respect of a plurality of training subjects derived from video images of the training subjects using the feature extractor.

Fig. 1

**Description**

The invention relates to methods of classifying a sleep state of a subject, in particular using machine-learning analysis of video images.

Polysomnography (PSG) is used in the diagnosis of various sleep-related disorders. During polysomnographic studies, subjects are monitored while sleeping, while various measurements of brain activity (the electroencephalogram - EEG), eye movement (electro-oculogram - EOG), and other physiological parameters such as the chin Electromyogram (EMG) are recorded. The resulting measurements can be used by a trained human scorer to determine the sleep stages, or other physiological events, undergone by the subject at various points during the period of monitoring, which in turn can be used for various purposes including the detection of medical conditions including insomnia, restless leg syndrome, and sleep apnoea.

Interpretation of the results of a polysomnography study requires review by a trained scorer. The scorer reviews (either on a computer screen or on a print-out) short 'epochs' of the measurements, for example 30-second epochs, and determines information including a classification of each epoch into one of a number of sleep stages. Typically, five stages are used: wake, rapid-eye movement (REM) sleep, and three categories of non-REM sleep indicating light, intermediate, and deep sleep. The latter are usually denoted N1, N2, and N3 in order of increasing depth of sleep. However, other divisions, particularly of non-REM sleep, are possible.

Interpretation by a trained scorer is time-consuming and labour-intensive, as several hours of measurements must be analysed in epochs of less than a minute each. Therefore, polysomnography studies are typically expensive, time-consuming, and difficult to arrange. In order to reduce the cost of sleep analysis and increase its availability for medical diagnosis, it would be desirable to develop systems for analysing sleep which do not require the full set of traditional PSG sensors and data, which often includes up to twelve channels of EEG, the two channels of EOG and the chin EMG, and replace them with more comfortable and less burdensome technology.

One example of an alternative technology is video monitoring. Unobtrusive sleep monitoring using video analysis could open the way for accessible and scalable sleep disorder screening and health monitoring. However, a lack of suitable video image data labelled with corresponding sleep states presents a challenge for effective training of machine-learning algorithms to identify sleep state from video images.

According to a first aspect of the invention, there is provided a method comprising determining a sleep state of a test subject using input data from the test subject, wherein: the input data comprise: at least one measure of test subject movement; and at least one cardiorespiratory feature of the test subject; the input data are derived from video images of the test subject; and the at least one cardiorespiratory feature of the test subject is derived using a feature extractor trained using reference cardiorespiratory signals for each of a plurality of reference subjects, the feature extractor comprising a neural network, and the reference cardiorespiratory signals derived from time-resolved measurements from a plurality of sensors worn by the reference subjects.

Using a feature extractor trained on cardiorespiratory signals from wearable sensors means that the feature extractor can be trained on large, pre-existing data sets from traditional PSG studies. This allows the feature extractor to be more effectively trained to identify features that are indicative of sleep state. This learning can then be transferred to the analysis of video images to improve the ability to identify sleep state from video images, thereby combatting the scarcity of labelled data for video image analysis of sleep state.

In some embodiments, the method further comprises training the feature extractor using the reference cardiorespiratory signals. In some embodiments, training the feature extractor comprises combining the feature extractor with a reference classifier to form a transfer network and training the transfer network to determine a sleep state for each of the reference subjects, optionally by training the transfer network to minimise a loss function, for example a cross-entropy loss. This is an effective way to train the feature extractor using labelled reference cardiorespiratory signals. A loss function such as a cross-entropy loss is a suitable function for quantifying the effectiveness of the algorithm during training.

In some embodiments, the reference cardiorespiratory signals in respect of each reference subject are temporally divided into a plurality of epochs, each epoch being labelled with one of a set of sleep states comprising wake, rapid-eye movement (REM) sleep, light non-rapid eye movement (NREM) sleep, or deep NREM sleep, optionally wherein in the set of sleep states further comprises intermediate NREM sleep. Labelling the reference cardiorespiratory signals with standard sleep stage labels means that the results of algorithms using the feature extractor are more easily compared with other sleep staging data and methods.

In some embodiments, the reference cardiorespiratory signals comprise one or more of heart rate, respiratory rate, a pulse waveform, and a respiratory waveform. These parameters can be gathered from analysis of video images and sensors worn by subjects and are indicative of the differences between the different sleep states.

In some embodiments, the reference cardiorespiratory signals comprise a first plurality of reference cardiorespiratory signals and a second plurality of reference cardiorespiratory signals, wherein the first and second pluralities of reference cardiorespiratory signals differ in one or more signal characteristics, optionally wherein the signal characteristics comprise sampling rate and duration. In some embodiments, the feature extractor extracts a first plurality of features from the first plurality of reference cardiorespiratory signals and a second plurality of features from the second plurality of reference cardiorespiratory signals. Using multiple sets of signals with different characteristics can enable the feature extractor to access additional information that make the resulting features better able to distinguish sleep states.

In some embodiments, the feature extractor combines the first and second pluralities of features into a plurality of output features, optionally wherein the plurality of output features is smaller than a total number of features in the first and second pluralities of features. Combining the features allows a single set of output features to be used by other algorithms and allows for information to be combined between the two set of features. Reducing the number of output features helps to reduce overfitting in the algorithms while still allowing for the feature extractor to take advantage of the different characteristics of the two sets of features.

In some embodiments, the method further comprises deriving the reference cardiorespiratory signals from the time-resolved measurements. This ensures that the reference cardiorespiratory signals are derived in a manner that is best compatible with the other parts of the method.

In some embodiments, deriving the reference cardiorespiratory signals from the time-resolved measurements comprises down sampling and/or filtering the time-resolved measurements. This allows the characteristics of the reference cardiorespiratory signals to be better matched to corresponding signals derived from video images, which in turn makes the features obtained by the feature extractor more transferrable to video image analysis.

In some embodiments, the method further comprises deriving the at least one cardiorespiratory feature of the test subject using the feature extractor. In some embodiments, the at least one cardiorespiratory feature of the test subject is derived from input cardiorespiratory signals using the feature extractor, optionally wherein the input cardiorespiratory signals comprise one or more of heart rate, respiratory rate, a pulse waveform, and a respiratory waveform. Deriving the features used for determining the sleep state from cardiorespiratory signals makes the process more analogous to that used to train the feature extractor, and therefore makes the derived features more effective at classifying sleep stages.

In some embodiments, the input cardiorespiratory signals comprise a first plurality of input cardiorespiratory signals and a second plurality of input cardiorespiratory signals, wherein the first and second pluralities of input cardiorespiratory signals differ in one or more signal characteristics, optionally wherein the signal characteristics comprise sampling rate and duration. Using multiple sets of signals with different characteristics can enable the feature extractor to access additional information that make the resulting features better able to distinguish sleep states.

In some embodiments, the input cardiorespiratory signals are derived from video images of the test subject, optionally wherein the method further comprises deriving the input cardiorespiratory signals from the video images of the test subjects. Deriving the input cardiorespiratory signals from the video image data, and then deriving the cardiorespiratory features from the signals using the feature extractor makes the process more analogous to that used to train the feature extractor, and therefore makes the derived features more effective at classifying sleep stages.

In some embodiments, the method further comprises deriving the at least one measure of test subject movement from the video images of the test subject. This ensures that the measure of subject movement is derived in a manner that is best compatible with the other parts of the method.

In some embodiments, the at least one measure of test subject movement comprises one or more of a measure of subject movement in a head region of the subject, a measure of movement in a torso region of the subject, and a measure of movement in an outer region of the video image. By breaking down the image into different regions, a greater level of detail can be obtained that is helpful in improving the accuracy of sleep state determination.

In some embodiments, determining the sleep state of the test subject comprises applying a machine-learning algorithm to the input data, wherein the machine-learning algorithm outputs a determination of the sleep state of the test subject on the basis of training data in respect of a plurality of training subjects; the training data are derived from video images of the training subjects; the training data comprise for each of the training subjects: at least one measure of subject movement; and at least one cardiorespiratory feature of the subject; and the at least one cardiorespiratory feature for each of the training subjects is derived using the feature extractor. Using a machine learning algorithm for the classification can allow the method to more effectively make use of the features obtained by the feature extractor, some of which may not be readily interpretable by a human.

In some embodiments, the machine learning algorithm is a regression algorithm, and the machine-learning algorithm determines the sleep state of the test subject using a continuous measure of sleep depth, optionally wherein the regression algorithm is a neural network. An advantage of machine-learning against traditional polysomnography techniques is that the sleep state can be quantified more precisely based on its similarity to the different traditional sleep labels.

In some embodiments, the training data in respect of each training subject are temporally divided into a plurality of epochs, each epoch being labelled with one of a set of sleep states; and the machine learning algorithm is a classification algorithm that classifies the sleep state of the test subject as one of the set of sleep states, optionally wherein the classification algorithm is one of a logistic regression, k-nearest neighbours, a random forest classifier, and a neural network. Classification of this type is more similar to the outcome of traditional polysomnography, and so can allow for more straightforward comparisons of the determined sleep states with previous data, or for diagnoses based on the determined sleep state using existing and traditional techniques.

In some embodiments, the training data in respect of each training subject are temporally divided into a plurality of epochs, each epoch being labelled with one of a set of sleep states; the machine learning algorithm is a classification algorithm, optionally one of a logistic regression, k-nearest neighbours, a random forest classifier, and a neural network; the classification algorithm generates scores for each sleep state of the set of sleep states, the scores representing a confidence of the sleep state of the test subject being that sleep state of the set of sleep states; and the machine-learning algorithm determines the sleep state of the test subject using a continuous measure of sleep depth, the continuous measure being determined from a weighted combination of the scores for each sleep state of the set of sleep states. This allows a continuous measure of sleep depth to be derived from the classification algorithms, which can give a more precise measure of the depth of sleep.

In some embodiments, the set of sleep states comprises wake, rapid-eye movement (REM) sleep, light non-rapid eye movement (NREM) sleep, or deep NREM sleep, optionally wherein in the set of sleep states further comprises intermediate NREM sleep. These correspond to the sleep states most commonly used in medical and scientific literature and so make the results of the method more comparable to other existing methods.

In some embodiments, the at least one cardiorespiratory feature for each of the training subjects is derived from training cardiorespiratory signals using the feature extractor, optionally wherein the training cardiorespiratory signals comprise one or more of heart rate, respiratory rate, a pulse waveform, and a respiratory waveform. In some embodiments, the method further comprises deriving the at least one cardiorespiratory feature for each of the training subjects from the training cardiorespiratory signals using the feature extractor. Deriving the features used for training from cardiorespiratory signals makes the process more analogous to that used to train the feature extractor, and therefore makes the feature extractor more transferrable to deriving the training data.

In some embodiments, the training cardiorespiratory signals comprise a first plurality of training cardiorespiratory signals and a second plurality of training cardiorespiratory signals, wherein the first and second pluralities of training cardiorespiratory signals differ in one or more signal characteristics, optionally wherein the signal characteristics comprise sampling rate and duration. Using multiple sets of signals with different characteristics can enable the feature extractor to access additional information that make the resulting features better able to distinguish sleep states.

In some embodiments, the training cardiorespiratory signals are derived from video images of the training subjects, optionally wherein the method further comprises deriving the training cardiorespiratory signals from the video images of the training subjects. Deriving the training cardiorespiratory signals from the video image data, and then deriving the cardiorespiratory features from the signals makes the process more analogous to that used to train the feature extractor, and therefore makes the feature extractor more transferrable to deriving the training data.

In some embodiments, the method further comprises deriving the at least one measure of subject movement from the video images of the training subjects. This ensures that the measure of subject movement is derived in a manner that is best compatible with the other parts of the method.

In some embodiments, the method further comprises training the machine-learning algorithm using the training data. This provides the method with the advantages as described for the training data.

In accordance with a second aspect of the invention, there is provided a method of training a machine-learning algorithm to determine a sleep state of a subject, wherein: the method uses training data in respect of a plurality of training subjects; the training data are derived from video images of the training subjects; the training data comprise for each of the training subjects: at least one measure of subject movement; and at least one cardiorespiratory feature of the subject; the at least one cardiorespiratory feature for each of the training subjects is derived using a feature extractor trained using reference cardiorespiratory signals for each of a plurality of reference subjects, the feature extractor comprising a neural network, and the reference cardiorespiratory signals derived from time-resolved measurements from a plurality of sensors worn by the reference subjects; and the method comprises training the machine learning algorithm using the training data.

Training the feature extractor on reference cardiorespiratory signals derived from sensors worn by subjects means that the feature extractor can be trained on large, pre-existing data sets from traditional PSG studies. This allows the feature extractor to be more effectively trained to identify features that are indicative of sleep state. This learning can then be transferred to the analysis of video images to improve the ability of the machine-learning algorithm to identify sleep state from video images, thereby combatting the scarcity of labelled data for training the machine-learning algorithm.

In some embodiments, the method further comprises deriving the at least one cardiorespiratory feature for each of the training subjects from training cardiorespiratory signals using the feature extractor, optionally wherein the training cardiorespiratory signals comprise one or more of heart rate, respiratory rate, a pulse waveform, and a respiratory waveform. In some embodiments, the method further comprises deriving the training cardiorespiratory signals from the video images of the training subjects. Deriving the features used for determining the sleep state from cardiorespiratory signals makes the process more analogous to that used to train the feature extractor, and therefore makes the derived features more effective at classifying sleep stages.

In some embodiments, the method further comprises deriving the at least one measure of subject movement from the video images of the training subjects. This ensures that the measure of subject movement is derived in a manner that is best compatible with the other parts of the method.

In some embodiments, the at least one measure of test subject movement comprises one or more of a measure of subject movement in a head region of the subject, measure of movement in a torso region of the subject, and a measure of movement in an outer region of the video image. By breaking down the image into different regions, a greater level of detail can be obtained that is helpful in improving the accuracy of sleep state determination.

The steps of the method may be carried out using a computer apparatus running an appropriate program, may be implemented by a dedicated hardware device, or may be implemented by any combination thereof. The computer apparatus, where used, may be any type of computer system but is typically of conventional construction. The computer program may be written in any suitable programming language. The computer program may be stored on a computer-readable storage medium, which may be of any type, for example: a recording medium which is insertable into a drive of the computing system and which may store information magnetically, optically or opto-magnetically; a fixed recording medium of the computer system such as a hard drive; or a computer memory. The data used by the method may be stored in a memory of the computer apparatus and/or hardware device in a conventional manner.

The invention may also be embodied in a computer program comprising, or a computer-readable storage medium having stored thereon, instructions which, when carried out by a computer, cause the computer to carry out the method of the first or second aspects. The invention may further be embodied in an apparatus configured to carry out the method of the first or second aspects.

Embodiments of the present invention will now be described by way of non-limitative example with reference to the accompanying drawings, in which:

Fig. 1 illustrates how transfer learning is applied to transfer learning from one dataset to another;

Fig. 2 is a flowchart of a method of training a machine-learning algorithm to determine a sleep state;

Fig. 3a) and Fig. 3b) illustrate how to calculate the angle between a camera and the vertical;

Fig. 4a) to Fig. 4c) show how a homography transformation can be applied to obtain a virtual viewpoint looking directly downward;

Fig. 5a) to Fig. 5d) illustrate the calculation of an optical flow field;

Fig. 6a) and Fig. 6b) illustrate segmentation of the video image into different regions;

Fig. 7a) and Fig. 7b) show a measure of subject movement derived from optical flow and an accelerometer signal, and the cross-correlation therebetween;

Fig. 8 shows examples of pulse and respiratory waveforms;

Fig. 9 is a flowchart of a method of determining a sleep state of a subject using a trained machine-learning algorithm;

Fig. 10 is a flowchart of a method of determining a sleep state of a subject using a machine-learning algorithm;

Fig. 11 is a flowchart of a method of training a feature extractor;

Fig. 12 shows an exemplary structure of the feature extractor and its inputs;

Fig. 13 shows further detail of the exemplary structure of the feature extractor;

Fig. 14 shows another exemplary structure of the feature extractor and its inputs;

Fig. 15 shows further detail of the second exemplary structure of the feature extractor;

Fig. 16 shows the Cohen's kappa distribution of a network used to train the feature extractor;

Fig. 17 shows an example hypnogram produced by the network used to train the feature extractor;

Fig. 18(a) and Fig. 18(b) show confusion matrices for five class sleep staging;

Fig. 19 shows a confusion matrix for wake-sleep classification using the transfer learning approach;

Fig. 20 shows a confusion matrix for three-class sleep staging using the transfer learning approach;

Fig. 21 shows a confusion matrix for four-class sleep staging using the transfer learning approach;

Fig. 22 shows a confusion matrix for five-class sleep staging using the transfer learning approach;

Fig. 23 shows violin plots of agreement between machine learning models and human scorers;

Fig. 24 shows a Bland-Altman analysis of agreement between a machine learning model using a feature engineering approach and human scorers;

Fig. 25 shows a Bland-Altman analysis of agreement between a machine learning model using the transfer learning approach and human scorers, as well as inter-scorer agreement;

Fig. 26 shows example hypnograms produced by human scorers and the machine-learning model using transfer learning for the same data;

Fig. 27 shows a confusion matrix for four-class sleep staging using the transfer learning approach in a second exemplary implementation;

Fig. 28 shows the distribution of Cohen's κ values as a function of age, sex, and Fitzpatrick skin type;

Fig. 29 shows an example sleep hypnogram generated from near-infrared video; and

Fig. 30 shows example four-class hypnograms during fragmented sleep.

To use video monitoring to analyse sleep stage progression requires identifying parameters that can be used to classify sleep state and that can be derived from video images.

The relationship between the activity of the autonomic nervous system and the progression of sleep stages through the night has long been established. Recent studies have attempted to leverage this association to estimate sleep parameters from the analysis of heart rate and respiratory rate measurements of autonomic activity derived from contact sensors worn by the subjects. While contact methods to monitor heart rate and activity establish the feasibility of autonomic sleep stage classification, these methods still require contact sensors, such as electrodes, finger probes, and accelerometers, to be attached to the body, which reduce comfort and thus limit prolonged use.

Recent success in estimating heart rate and respiratory rate in a non-contact manner using new modalities such as video cameras, radar, or Wi-Fi sensors, suggests that it may be possible to combine video monitoring with the insight that heart rate and respiratory rate measurements of autonomic activity are indicative of sleep progression to develop non-contact methods for sleep staging. This is particularly useful in settings where wearable devices are unsuitable.

The methods described herein use features including cardiorespiratory (CR) features 8 and measures of movement 6 to determine the sleep states of a subject. Traditionally, these features would be engineered by a human designer to choose features known to be indicative of sleep state, such as heart rate, respiration rate, the variances of heart rate and respiration rate, and so on. However, such so-called feature engineering methods suffer from a number of limitations. For example, simple statistics such as means and standard deviations are not robust to noise and discard valuable information for sleep stage classification.

For this reason, existing methods for estimating sleep parameters from the analysis of heart rate and respiratory rate measurements often make use of machine learning models to infer sleep stages based on features of these signals. Machine learning-based feature extractors, such as convolutional neural networks (CNNs), can overcome the disadvantages of feature engineering methods by learning suitable features to represent the data directly from time series data. This has been crucial to the success of deep learning previously demonstrated in other problem domains such as image recognition.

However, the training of state-of-the-art supervised deep learning architectures is data-intensive. Previous work in automated sleep staging using deep learning has typically used datasets for traditional PSG studies using contact-based sensors, such as the Sleep Heart Health Study (SHHS), which are larger and more widely available. As non-contact methods are not included in standard PSG data sets, collecting the large data sets required for training models on both input data from non-contact methods and gold-standard sleep parameters (which necessitate PSG) is resource-expensive. Therefore, data scarcity for training state-of-the-art neural networks based on non-contact methods has been a challenge for developing non-contact sleep monitoring technologies.

The present invention overcomes this limitation by leveraging knowledge from a feature extractor trained on a large but comparable reference data set to solve the problem on a target training data set where fewer data samples are available for training. Deep neural networks can learn intermediate representations that are transferable to other tasks [1]. By training a feature extractor on a large, source dataset and fine tuning it on a smaller target dataset, the present method can achieve greater performance than training the feature extractor directly on the target dataset, which can lead to over-fitting dependent on the relative size of the datasets. Transfer learning has previously been used in the sleep staging work of Radha et al., who train a classification model using features derived from the ECG and fine tune it on a smaller dataset using features derived from PPG signals [6]. They show that this leads to better performance than training a model solely on the smaller dataset of PPG signals.

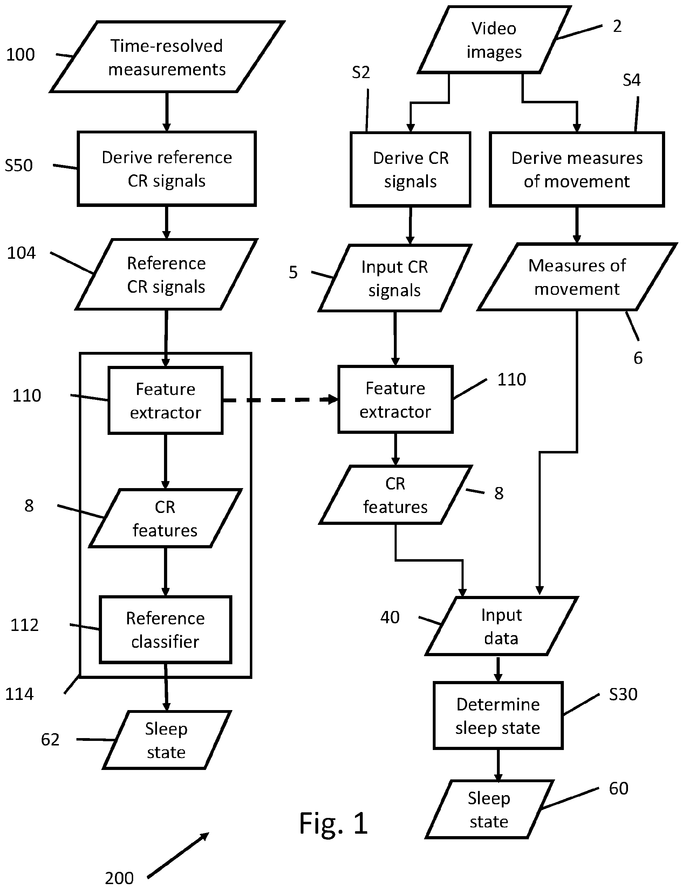

Fig. 1 illustrates schematically how the feature extractor 110 is trained and transferred to be used in determining a sleep state. The feature extractor is first pre-trained using a large data set of reference physiological measurements obtained in traditional polysomnographic studies with contact sensors. Once this model is trained, it is applied to corresponding physiological signals obtained from video images to obtain the feature values that are used as inputs to classification models in order to determine the sleep state of a subject via a non-contact method.

One or more additional inputs obtained from the video images 2 (and not available in the data set obtained from contact sensors) are also used in the classification model, including measures of subject movement 6 and one or more other measures such as body position, to fine-tune the model to further improve performance. Body position may include, for example, a binary measure of whether the subject is resting in bed. This is known as the fine-tuning phase. In the fine-tuning phase, the feature extractor 110 components are adjusted to the video-image domain.

As shown on the left-hand side of Fig. 1, the feature extractor 110 is trained by combining the feature extractor 110 with a reference classifier 112 to form a transfer network 114. Reference CR signals 104 derived S50 from time-resolved measurements 100 from sensors worn by a plurality of reference subjects are fed into the transfer network 114. Within the transfer network 114, the CR features 8 from the penultimate layer of the feature extractor 110 are fed to the reference classifier 112.

The time-resolved measurements 100 are labelled with sleep states, for example by human scorers. Using these labels, the transfer network 114 is trained by supervised training to determine a sleep state 62 for each of the reference subjects from the reference CR signals 104. The CR features 8 derived from the feature extractor 110 are then expected to encode information which makes the sleep stages linearly separable and thus to be effective when used as inputs to an alternative sleep stage classification model.

After training the transfer network 114, the trained feature extractor 110 is used as the feature extractor 110 to obtain the CR features 8 from a video image 2 dataset from a test subject in the steps on the right-hand side of Fig. 1. The CR features 8 are combined with the motion-derived measures of subject movement 6 to form input data 40 that are used to determine S30 the sleep state 60 of a test subject from video images 2 of the test subj ect.

The structure of the feature extractor and how it is trained are discussed in more detail below. Using transfer learning allows the method to learn cardiorespiratory features directly from the time-resolved measurements, leveraging the representational capacity of deep neural networks without requiring the volume of data typically required for supervised learning.

Determining S30 the sleep state of the test subject may be carried out using a human-designed algorithm that accepts the input data 40 and outputs a determination of the sleep state 60. Alternatively, determining S30 the sleep state of the test subject may comprise applying a machine-learning algorithm to the input data 40.

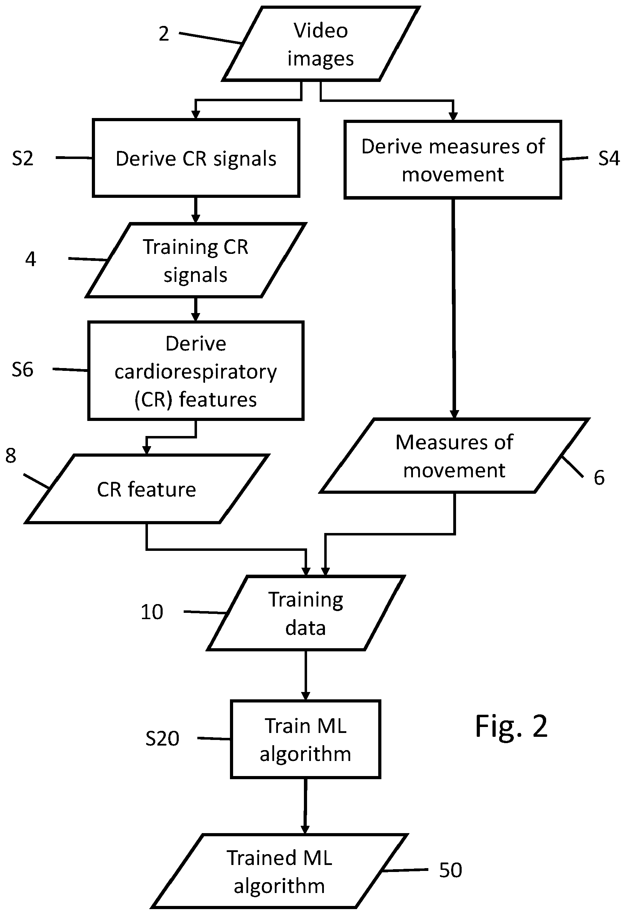

Fig. 2 is a flowchart showing a method of training a machine-learning algorithm to determine a sleep state of a subject. The method uses training data 10 in respect of a plurality of training subjects. The training data 10 are derived from video images 2 of the training subjects.

The video images 2 can be captured using a conventional visible-light or near-infrared (e.g. 850nm) camera, such as may be used for video surveillance. Vital sign characteristics can be extracted from the video images by detecting signals from changes in colour of a subject's skin and/or movements of the subject's body. These signals are indicative of changes in blood flow and movements of the chest during breathing. Various methods for extracting respiratory rates and heart rates from video images of a subject are known in the art and may be used in the present methods. An example is the Oxevision system provided by Oxehealth Ltd, and the techniques discussed in European patent application No. 19220087.1.

For clarity it should be noted that the heart rates and respiratory rates referred to herein (whether derived from video images or from a plurality of sensors worn by subjects) are time-resolved signals comprising values of heart rate or respiratory rate for each of a plurality of points in time. This allows the changes in the heart rate or respiratory rate over time to be taken into account.

The training data 10 in respect of each training subject are temporally divided into a plurality of epochs. These epochs are generally of equal length, typically 30 seconds, although this is not essential and other lengths of epoch may be used.

The training data 10 comprise for each of the training subjects at least one feature derived from the video images of the subjects. The at least one feature comprises at least one measure of subject movement 6 and at least one cardiorespiratory feature 8 of the subject. The features may be derived directly from the video images, or may be derived from time-resolved measurements that are themselves derived from the video images.

In particular, the at least one cardiorespiratory feature for each of the training subjects is derived from training cardiorespiratory signals 4 using the feature extractor. As will be discussed in more detail below, the training cardiorespiratory signals 4 comprise one or more of heart rate, respiratory rate, a pulse waveform, and a respiratory waveform, and may comprise other signals as well.

The method comprises deriving the training data 10 from the video images 2 of the subjects. However, in other embodiments, the training data 10 may be derived from the video images 2 prior to training the machine-learning algorithm, such that the step of deriving the training data 10 is not a part of the method of training.

The training data 10 may be derived in various ways. The training data 10 may be fully or partially derived using a machine-learning algorithm. In particular, the method comprises deriving the at least one cardiorespiratory feature 8 from the training cardiorespiratory signals using the feature extractor 110. In this situation, the deriving of the training data 10 and the determining S30 of a sleep state of the subject 60 may be carried out by separate machine-learning algorithms. Alternatively, the deriving of the training data 10 and the determining S30 of a sleep state 60 of the subject may be performed together by a combined machine-learning algorithm, such as a neural network. In the discussion of determining sleep state that follows, references to the 'machine-learning algorithm' will generally refer (unless identified otherwise explicitly or by context) to the machine learning algorithm or part of the combined machine-learning algorithm that determines S30 the sleep state 60 of the subject. For example, in the case of a combined machine-learning algorithm in the form of a neural network, this could refer to the last stage classification layer or layers.

Deriving the training data 10 comprises deriving S4 the at least one measure of subject movement 6 from the video images 2 of the training subjects. The measures of subject movement 6 may comprise a measure of subject movement in a head region of the subject and/or a measure of movement in a torso region of the subject.

The measure of the movement 6 in a head region of the subject is derived S4 from the video images 2 by segmenting a region of the video frame that is known or likely to contain the subject's head, for example the head region of the bed in which the subject is sleeping. The head region may be segmented from a rectified projection of a reference image frame. The measure of movement 6 in a torso region of the subject can be derived S4 using the same process, but with the image segmented so as to cover a region over the region of the image known or likely to contain the subject's torso.

The measures of subject movement 6 may further comprise a measure of movement in an outer region of the video image 2. The measure of movement in the outer region may be derived S4 from the video images 2 by segmenting an outer region of the video frame that is not expected to routinely contain the subject. For example, the outer region may be a region of the video frame outside (i.e. not including) the bed in which the subject is sleeping. The outer region may be located substantially at or around some or all of the outer edge of the video frame. The outer region may surround, but not intersect or overlap with, one or both of the region of the video frame that is known or likely to contain the subject's head and the region of the image known or likely to contain the subject's torso.

In a clinical setting bed and camera positions are likely to vary, therefore it is important that any system-in-use is invariant to changes in either of these parameters. An image homography can be used to improve the robustness of the method to the camera angle.

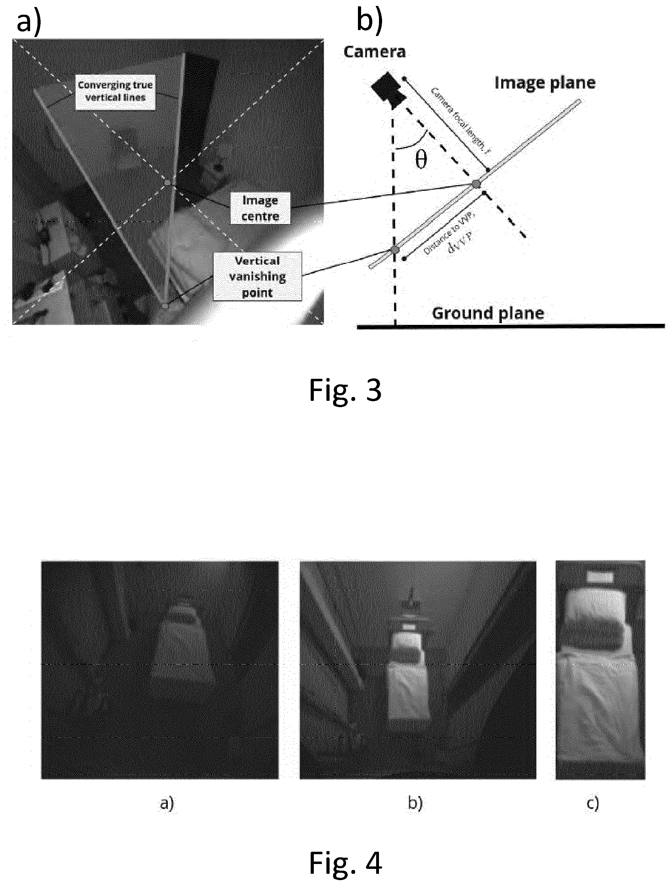

Using a still image and knowledge of the video camera parameters including its focal length it is possible to calculate the angle between the camera and the vertical. This process is illustrated in Fig. 3. The vertical vanishing point and camera angle from the vertical can be calculated. The vertical vanishing point (VVP) is calculated by finding the convergence point of true vertical lines in the image such as wall edges. The focal length and distance to the VVP in the image plane can be determined from the camera parameters and pixel co-ordinates. This allows the angle between the camera and the vertical $\theta$, to be calculated using simple trigonometry.

Using the camera angle, a homography transformation is applied to obtain a virtual viewpoint looking directly downward. A rotation about the vertical axis is also applied such that the bed is aligned to the vertical. After applying the transformation the images can be cropped to the bed region for further video processing. This process is illustrated in Fig. 4. Camera angle invariance is achieved using image rectification. Fig. 4a) shows an example frame. In Fig. 4b), the frame is rectified using a homography transformation. In Fig. 4c) the frame is cropped to the bed region for further processing

To derive S4 the measure of subject movement 6, an image optical flow signal is computed at a suitable frequency (for example 1-10 Hz, preferably 3-6 Hz, most preferably 5 Hz) over the segmented head region. Optical flow is the apparent motion of pixels in the image plane between frames. Sparse optical flow methods track the movement of a small number of points in the images over time, while dense optical flow methods compute a vector field over the entire image which indicate the movement of each pixel between frames.

From the cropped and rectified view of Fig. 4, the dense optical flow field over the bed region can be calculated between frames up to the frame rate of the camera (e.g. 20 FPS). The algorithm used here for calculating dense optical flow field is the Dense Inverse Search algorithm, because it has low computational complexity and high accuracy, but in general any suitable algorithm can be used.

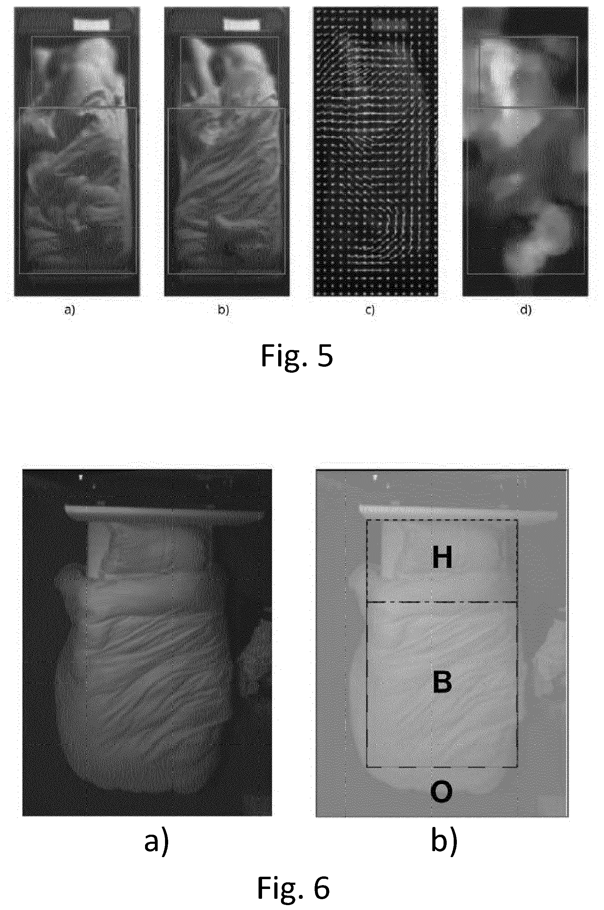

Fig. 5 shows an example optical flow field captured during a small change in body position during sleep. Fig. 5a) and Fig. 5b) show two frames captured one second apart. Fig. 5c) shows the resulting optical flow field and Fig. 5d) shows the corresponding magnitude image. The participant's face has been blurred.

From the optical flow field it is possible to calculate a continuous time series measure of subject movement 6. A simple method for doing this is to use the spatial average of the magnitude of the flow field. This idea can be extended by calculating separate measures for the head and torso regions as mentioned above. This retains some information about the spatial extent of movement. Both of these measures are calculated at a suitable frequency, e.g. 5Hz. The frequency is chosen as a trade-off between temporal resolution and computation time. The head region (red) and torso region (blue) bounding boxes, used to derive the corresponding measures of subject movement are plotted in Fig. 5. The head region may cover approximately the upper third of the rectified view, and the torso region the lower two thirds. The bounding boxes in Fig. 5 are manually specified, but the bounding boxes may also be automatically identified, for example using deep learning approaches.

Fig. 6 illustrates an embodiment in which the measures of subject movement 6 further comprise a measure of movement in an outer region of the video image 2. As shown in Fig. 6b), the rectified video frame in Fig. 6a) is segmented to include head (H) and torso (B) regions, and is further segmented to include an outer region (O) that surrounds the head and torso regions.

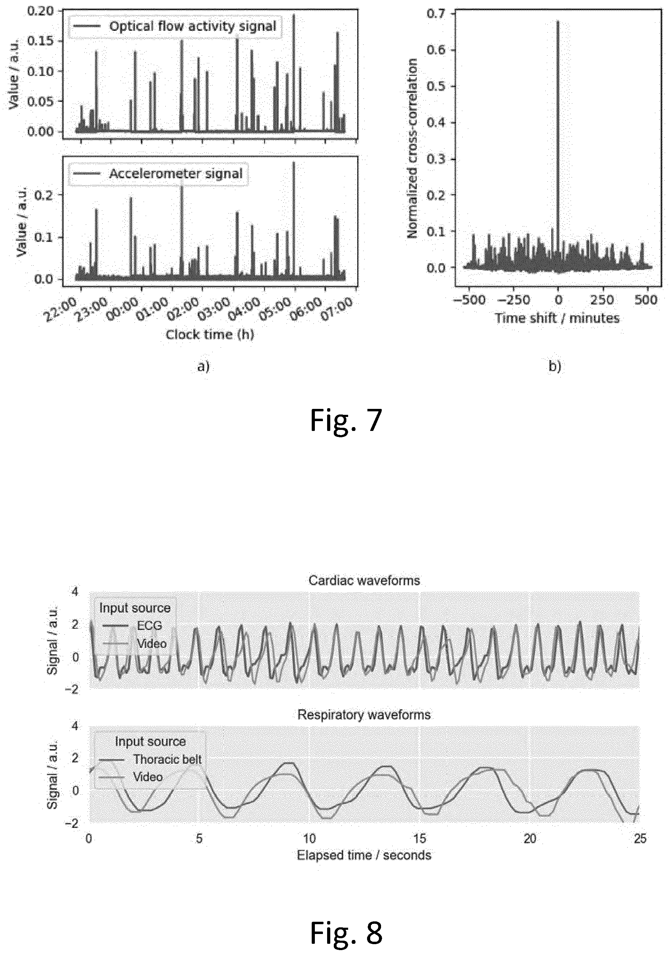

Fig. 7a) shows a measure of subject movement 6 derived from optical flow, and also shows an accelerometer signal recorded at the same time as the video image from which the optical flow is determined. The source of the accelerometer signal is a PSG recording device attached to the patient's chest. The optical flow signal uses the average magnitude of the flow field in the torso region. Fig. 7b) shows the normalised cross-correlation between the two signals. Cross-correlation measures the similarity between two time series as a function of the relative shift between them. Fig. 7b) shows a sharp peak in the cross-correlation for zero time shift, indicating there is good correlation between the two signals.

The magnitude of the optical flow signal is processed by subtracting the mean value for each recording, and filtering the subtracted signal using a Gaussian smoothing filter (e.g. with σ = 600 seconds). The resulting filtered signal may then be downsampled by interpolation at a lower frequency (e.g. less than 5 Hz, preferably 1 Hz). Further normalisation may be performed by a suitable reference value, for example the 90th percentile value for that recording. For a given epoch, the measure of the movement in the head region of the subject and the measure of movement in the torso region of the subject are calculated as an average (e.g. the mean) of the values of the measure in a time window centred on that epoch. The time window may have any suitable length, for example 10 minutes. This particular process of extracting the measure is not essential, however, and any other suitable process may be used that results in a quantity representative of the subject's movement in a head region.

For both measures of movement 6, values may still be returned even where the subject is not in the bed, and therefore not within the segmented regions corresponding to the head region and torso region. To avoid distorting the training of the machine-learning algorithm, a further feature may be included indicating a binary value of whether the subject is considered to be in bed. This could be determined by conventional image recognition techniques used to detect the presence of a subject in the corresponding areas of the video image.

For either measure of movement 6, pre-processing may be used to reduce or remove drift in the baseline signal (also known as baseline wander), and reduce or remove spurious peaks. The signal may also be calibrated based on an initial wake period of the recording of the subject's sleep opportunity, i.e. the period before the subject has initially fallen asleep.

The measures of movement 6 may comprise multiple different measures of similar quantities calculated using different characteristics in order to allow the machine-learning algorithm to take account of information that is encoded in different characteristics, such as being averaged over different spatial regions and/or time scales. For example, plural measures of subject movement in a head region and/or plural measures of movement in a torso region of the subject may be used having different characteristics.

Other measures derived from the measures of subject movement in a head region or torso region may be also be used. For example, the measures of subject movement may comprise a measure of the time since subject activity was above a predetermined threshold for at least a predetermined time, where subject activity can be quantified with optical flow or another similar measure. It is also possible to use a neural network to extract measures of subject movement directly from the optical flow data or the video images 2.

An example set of 20 measures of movement 6 is shown in Table 1. These measures broadly fall into two categories: integral features, which quantify the amount of motion that occurred around the epoch, and counter-based features, which quantify the elapsed time since movement occurred. In general, integral features may be calculated as an average of the values of optical flow activity in a time window centred on the epoch, and counter-based features may be calculated based on a time since the optical flow signal was above a threshold for at least a predetermined time. The counter-based features in Table 1 are designed to encode for the rate of body movements, which are known to correlate strongly with sleep stages. In the example set in Table 1, features are computed for the upper and lower bed regions (i.e. the measures of movement in the head and torso regions respectively) separately.

**Table 1**

| Count | Feature description |
|---|---|
| **14** | **Integral features** |
| 6 | 30-second Gaussian-weighted sums, time-shifted by (-30, 0.0, 30.0) seconds. |
| 6 | 180-second Gaussian-weighted sums, time-shifted by (-180.0, 0.0, 180.0) seconds. |
| 2 | 20-minute Gaussian weighted sums. |
| **6** | **Counter-based features** |
| 4 | Time elapsed since activity signal was above a threshold $\in$ {0.1, 1.0}. |
| 2 | Time elapsed since activity signal was above a threshold (1.0) for 3 seconds. |

The measures of movement 6 may be calculated based on movement signals calculated for each region of the video image 2, the movement signals characterising subject movement within the region. For example, for each region of the video image 2 (e.g. head, torso, and outer), two movement signals may be calculated corresponding to a maximum magnitude of movement within the region and a total amount of movement within the region at each point in time. The movement signals may be calculated using the optical flow data.

For example, for each region R of the two-dimensional optical flow field $\boldsymbol{u}(t, x, y) \in \mathbb{R}$ calculated for the

video image 2, the movement signals can be calculated as

$$v(t; R) = \max_{(x,y)\in R} |u(t,x,y)| \qquad (0.1)$$

$$s(t; R) = \frac{1}{|R|} \sum_{(x,y)\in R} |u(t,x,y)| \qquad (0.2)$$

where $v(t; R)$ corresponds to the maximum optical flow magnitude within the region at time $t$, and $s(t; R)$ corresponds to the average of the optical flow magnitude within the region R at time $t$.

Using these movement signals, the measures of movement 6 can be calculated. Examples of measures of movement 6 include measures that encode for motion within a time window $\Delta$ up to time t, such as

$$f_1(t; R, \Delta) = \sum_{t-\Delta}^{t} v(t; R) \qquad (0.3)$$

$$f_2(t; R, \Delta) = \sum_{t-\Delta}^{t} s(t; R) \qquad (0.4)$$

These measures correspond to various measures of the motion characteristics, specifically:

- the sum ($f_1$) of the maximum optical flow magnitude in the region at each time point within the time window,
- the sum ($f_2$) of the optical flow magnitude over the entire region within the time window,.

The measures of movement may also include measures of the time elapsed since either of the movement signals went above a predetermined threshold. Examples include:

$$f_3(t; R, \delta) = \sum_{t-\tau}^{t} 1$$
$$\text{where} \quad \tau = \max_{t' \leq t} t'$$
$$\text{s.t.} \quad v(t'; R) > \delta \qquad (0.5)$$

$$f_4(t; R, \delta) = \sum_{t-\tau}^{t} 1$$
$$\text{where} \quad \tau = \max_{t' \leq t} t'$$
$$\text{s.t.} \quad s(t'; R) > \delta \qquad (0.6)$$

As in Table 1 above, a variety of measures of subject movement 6 can be included for different values of the time window $\Delta$ and/or the threshold $\delta$.

Two further measures of movement that could be used are the following:

$$f_a(t; R, \Delta) = \max_{t' \in [t-\Delta, t]} v(t'; R) \qquad (0.7)$$

$$f_b(t; R, \Delta) = \max_{t' \in [t-\Delta, t]} s(t'; R) \qquad (0.8)$$

These correspond to:

- $f_a$: the maximum optical flow magnitude in the region within the time window, and
- $f_b$: the maximum total optical flow in the region within the time window.

These measures are not used in the example results discussed below, but may provide additional information in some embodiments.

While optical flow is used in these specific examples, other measures of subject activity in the regions of the video image 2 could also be used to determine corresponding movements signals and measures of subject movement.

As mentioned above, the at least one cardiorespiratory feature for each of the training subjects is derived from training cardiorespiratory signals 4 using the feature extractor. The training cardiorespiratory signals are derived from the video images 2 of the training subjects.

Deriving the training data 10 further comprises deriving S2 the training cardiorespiratory signals 4 from the video images 2 of the training subjects. The at least one cardiorespiratory feature 8 is then derived S6 from the training cardiorespiratory signals 4.

To improve the consistency of the data and reduce the impact of spurious measurements, it is preferable that the training cardiorespiratory signals 4 for each epoch are calculated as an average (e.g. the mean) of the relevant values in a time window centred on that epoch. The time window may have any suitable length, for example 10 minutes.

The training cardiorespiratory signals 4 comprise heart rate (HR) and respiratory rate (RR). As mentioned above, the respiratory rate (RR, also referred to as breathing rate or respiration rate) can be extracted from the video images 2 by various known techniques. Respiration causes movements of the chest wall which can be measured to calculate a rate of respiration through methods such as frame-differencing, using dense optical flow magnitudes or by tracking the movement of sparse optical flow vectors in the video sequence.

The heart rate (HR) can also be derived from the video images 2 by various known techniques. Pulses of blood caused by heart beats cause fluctuations in the colour of a subject's skin, caused by the variation in blood oxygenation over the cardiac cycle and often referred to as "micro-blushes", and which can be detected by a camera. Heart rate can also be detected from head and body movements, caused by the ballistic forces generated as the heart pumps blood through the aorta. The measurement of these movements is known as ballistocardiography.

The training cardiorespiratory signals 4 may further comprise a pulse waveform and/or a respiratory waveform. In the case of the training cardiorespiratory signals 4, the pulse waveform and respiratory waveform are derived from the video images 2.

The pulse waveform is a time-resolved waveform representative of the subject's pulse or cardiac activity, and may be used to derive the heart rate. The pulse waveform may be representative of the magnitude or amplitude of the skin colour fluctuations or ballistocardiographic movements detected by the camera. The pulse waveform will typically have higher time resolution than the heart rate. For example, the heart rate may have a time resolution of approximately 1 Hz, while the pulse waveform may have a time resolution of approximately 10 Hz.

The respiratory waveform is a time-resolved waveform representative of the subject's breathing, and may be used to derive the respiratory rate. The respiratory waveform may be representative of the magnitude or amplitude of movement of the subject's chest as detected by the camera. The respiratory waveform will typically have higher time resolution than the respiratory rate. For example, the respiratory rate may have a time resolution of approximately 1 Hz, while the respiratory waveform may have a time resolution of approximately 5 Hz.

Examples of the pulse (cardiac) waveform and respiratory waveform are shown in Fig. 8. Using waveforms rather than HR and RR makes more data available to the machine learning algorithm for determining sleep state. This can allow the feature extractor to make use of other aspects of the data in predicting sleep states that may not be obviously relevant to a human observer.

For any of the input features (cardiorespiratory features 8 or measures of subject movement 6), the values used to calculate the overall feature value for an epoch may also be weighted using a confidence metric. In some systems used to extract the training data 10 from video images, a value may be provided for a confidence in each value. The values may then be weighted by the confidence value when calculating an overall average for the epoch, and/or may be excluded entirely if the confidence is considered too low (i.e. falls below a predetermined threshold such as 50% confidence). This can help to reduce the impact of spurious signals.

As well as the values for the various features derived from the video images 2 and making up the training data 10 discussed above, each epoch in the training data 10 is labelled with one of a set of sleep states. The set of sleep states comprises wake, rapid-eye movement (REM) sleep, light non-rapid eye movement (NREM) sleep, or deep NREM sleep. In what follows, epochs labelled as wake may be referred to as wake epochs, epochs labelled as light NREM sleep may be referred to as light NREM epochs, and so on for the other sleep states. These sleep states may also be referred to using their designations under the commonly-used sleep scoring system used by the American Academy of Sleep Medicine (AASM), which are W, REM, N1, and N3 respectively.

The labelling of the epochs will typically be obtained from interpretation by a trained polysomnography scorer of the training data and/or other data collected at the same time as the video images from which the training data are derived (for example from sensors worn by the subject). This labelling establishes definitions of the 'true' sleep state for each epoch against which the machine-learning algorithm can be trained in a supervised manner.

The set of sleep states may further comprise intermediate NREM sleep. Polysomnography studies typically label an intermediate NREM sleep state in addition to the light and deep NREM sleep states. Intermediate (or medium) NREM sleep may be referred to as N2 sleep under the system used by the AASM. Intermediate NREM sleep can be difficult to distinguish from light and deep NREM sleep, because of its similarities to both other sleep depths. Nonetheless, these intermediate NREM sleep epochs can provide valuable additional data for training the machine-learning algorithm.

The method comprises training S20 the machine learning algorithm using the training data 10. The machine-learning algorithm may be trained and tested using any suitable method, for example leave-one-out cross-validation on a per-night basis. The epochs of training data 10 used for training S20 the machine-learning algorithm may be limited to improve performance. For example, only epochs for which the agreement between human scorers evaluated exceeds a predetermined threshold may be used to train S20 the machine learning algorithm, such as where 2 or 3 human scorers agree on the applicable sleep stage label.

Various different types of machine-learning algorithm may be used for the determination of the sleep state, depending on how the determined sleep state is to be used. The machine learning algorithm may be a regression algorithm, for example a neural network. An example of a suitable neural network for the regression algorithm is a multilayer perceptron regressor. In this case, the machine-learning algorithm determines the sleep state using a continuous measure of sleep depth. The continuous measure is preferably normalised so that it can be easily compared between different subjects. For example, the continuous measure may be a number between -1 and +1, where a value of +1 represents wake and a value of -1 represents deep sleep. The particular range of values chosen as the scale for sleep depth is not particularly limited. For example, in other embodiments, a value of 0 may represent wake and a value of 1 may represent deep sleep.

The machine learning algorithm may be a classification algorithm, for example one of a logistic regression, k-nearest neighbours, a random forest classifier, and a neural network. An example of a suitable neural network is a neural multilayer perceptron. A random forest classifier is less prone to overfitting than a neural network in the low data regime, so may be preferred in such situations and may allow more features from the training data 10 to be used simultaneously without observing strong overfitting. A Random Forest is also better able to handle missing data, so may be preferred where data are incomplete. In this case, the machine-learning algorithm may determine the sleep state to be one of the set of sleep states.

Where the machine-learning algorithm is a classification algorithm, it may still output a numerical value (or score) for each of the sleep states. In such embodiments, the classification algorithm generates scores for each sleep state of the set of sleep states. The scores represent a confidence of the sleep state being that sleep state of the set of sleep states.

The ultimate determination of the sleep state as a classification into one of the sleep states indicated by the training labels may be determined from these scores in various ways, for example by choosing the sleep state associated with the training label having the highest classification score. However, the scores themselves can also be used to calculate measures of the sleep depth. For example, the supervised machine-learning algorithm may determine the sleep state using a continuous measure of sleep depth, where the continuous measure is determined from a weighted combination of the scores for each sleep state of the set of sleep states. To aid in interpretability and comparison between subjects, the scores are preferably normalised such that the sum of the scores is equal to a predetermined value, e.g. 1.0. The scores can then be combined into a continuous measure of sleep depth in any suitable manner depending on factors such as the desired range of values for the measure of the sleep state.

As mentioned above, a method comprising determining S30 the sleep state 60 of the test subject may be carried out using a human-designed algorithm or by applying a machine-learning algorithm to the input data 40.

The input data 40 comprise: at least one measure of test subject movement; and at least one cardiorespiratory feature of the test subject. Similarly as for the training data 10, the input data 40 are derived from video images 2 of the test subject. The method comprises deriving the input data 40 from the video images 2 of the test subject. However, in other embodiments, the input data 40 may be derived from the video images 2 prior to determining the sleep state, such that the step of deriving the input data 40 is not a part of the method. The video images 2 can be analysed to obtain the input data 40 for the test subject in the same way as described above for obtaining the training data 10 from the video images 2 of the training subjects. Also as discussed above for the training data 10, the input data 40 may be fully or partially derived using a machine-learning algorithm. In particular, the at least one cardiorespiratory feature 8 of the test subject is derived using the feature extractor 110.

Deriving the input data 40 comprises deriving S6 the at least one cardiorespiratory feature 8 of the test subject from input cardiorespiratory signals 5 using the feature extractor.

The input cardiorespiratory signals 5 comprise heart rate and respiratory rate. The input cardiorespiratory signals 5 may further comprise a pulse waveform and/or a respiratory waveform. The input cardiorespiratory signals 5 are derived S2 from video images 2 of the test subject in a similar manner as described for the training cardiorespiratory signals 4 discussed above.

The input data 40 are then composed of the derived cardiorespiratory features 8 and measures of movement 6 for the test subject. Deriving the input data 40 comprises further comprises deriving S4 the at least one measure of test subject movement 6 from the video images 2 of the test subject.

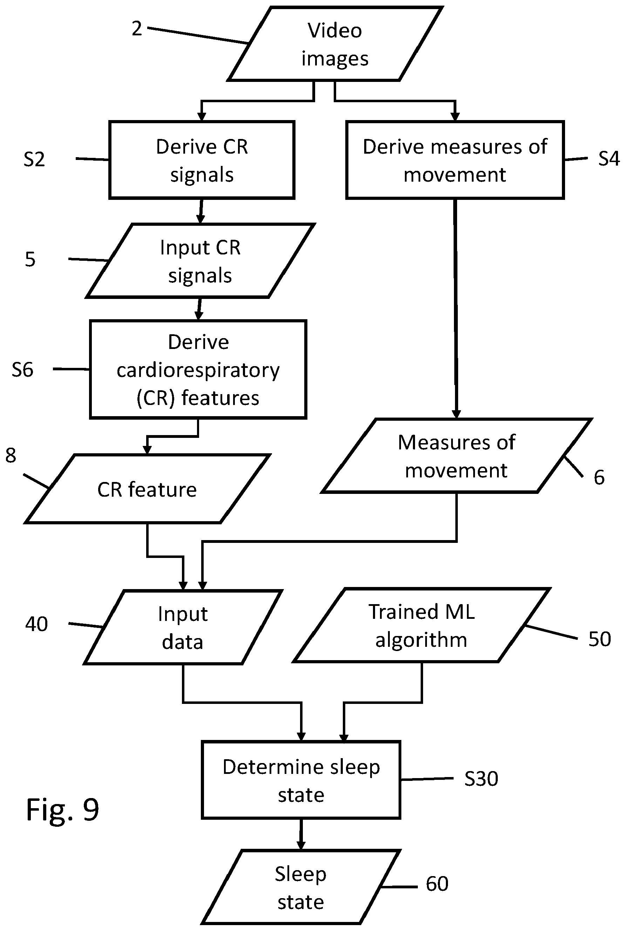

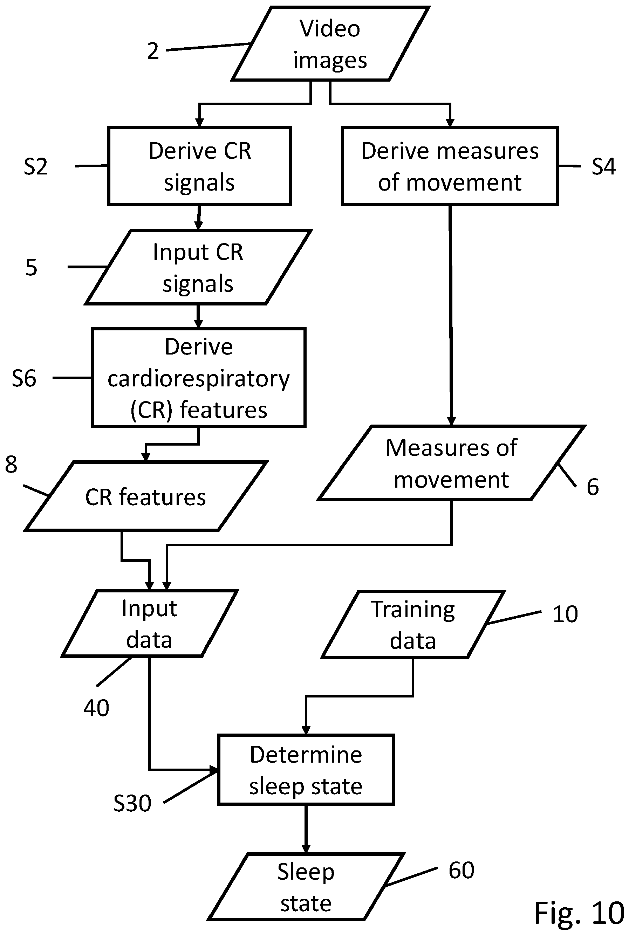

Fig. 9 and Fig. 10 show example methods in which the sleep state 60 of the test subject is determined by applying a machine-learning algorithm to the input data. The methods in Fig. 9 and Fig. 10 comprise deriving the input data 40 from the video images 2 of the test subjects, but as mentioned above, in some embodiments this may be performed separately beforehand.

The machine-learning algorithm outputs a determination of the sleep state 60 of the test subject on the basis of training data 10. The training data 10 are in respect of a plurality of training subjects and are derived from video images 2 of the training subjects, as described above. The training data 10 and training labels 30 are otherwise as described above in relation to the training methods. In particular, the at least one cardiorespiratory feature 8 for each of the training subjects is derived using the feature extractor 110.

The deriving of the input data 40 and the determining S30 of a sleep state 60 of the subject may be carried out by separate machine-learning algorithms or by a combined machine-learning algorithm, such as a neural network. In the discussion herein, references to the 'machine-learning algorithm' will generally refer (unless identified otherwise explicitly or by context) to the machine learning algorithm or part of the combined machine-learning algorithm that determines the sleep state of the subject.

There are two main options for the determination of the sleep state 60 of the test subject on the basis of the training data 10 using a machine-learning algorithm. Which of these is used will depend on the type of machine-learning algorithm that is being used.

The first option is shown in Fig. 9, where the machine-learning algorithm is trained using a method of training a supervised machine learning algorithm, such as that discussed above. In this case, the machine-learning algorithm is pre-trained, and the input data 40 and trained machine-learning (ML) algorithm 50 are used together to determine S30 the sleep state 60. The input data 40 are fed into the trained machine-learning algorithm 50, which outputs the determination of the sleep state 60. Neural networks such as the multilayer perceptron (whether as a regressor or classifier) are an example of this type of machine-learning algorithm. The input to step S30 corresponding to the trained ML algorithm 50 may comprise a plurality of parameters that were determined by the method of training the machine-learning algorithm.

The second option is shown in Fig. 10, where the machine-learning algorithm is of a type which is not pre-trained, such as a k-nearest neighbours classifier, but compares the input data 40 to the training data 10 at the time of being run. Again, the video images 2 of the test subject are used to derive the input data 40 as described above. In this case, the relabelling S10 of the epochs in the training data 10 is carried out during the method, and the training data 10 are required to determine the sleep state for subsequent new input data 40 from test subjects. The training data 10 may also be derived from the video images 2 of the training subjects at the time of carrying out the method using the steps described above in connection with Fig. 2. However, preferably, and as shown in Fig. 10, the training data 10 are derived from the video images 2 of the training subjects at an earlier time and provided at the time of carrying out the method. Since the training data 10 do not change for different test subjects, this reduces computational requirements by reducing the amount of processing that must be carried out for each new test subject.

Similarly as discussed above in relation to the methods of training, various different types of machine-learning algorithm may be used for the determination of the sleep state, depending on how the determined sleep state is to be used.

The machine learning algorithm may be a regression algorithm, for example a neural network. An example of a suitable neural network for the regression algorithm is a multilayer perceptron regressor. In this case, the machine-learning algorithm determines the sleep state of the test subject using a continuous measure of sleep depth.

The machine learning algorithm may be a classification algorithm, for example one of a logistic regression, k-nearest neighbours, a random forest classifier, and a neural network. An example of a suitable neural network is a neural multilayer perceptron. In this case, the machine-learning algorithm may determine the sleep state of the test subject to be one of the set of sleep states.

Where the machine-learning algorithm is a classification algorithm, it may still output a numerical value (or score) for each of the sleep states. In such embodiments, the classification algorithm generates scores for each sleep state of the set of sleep states. The scores represent a confidence of the sleep state being that sleep state of the set of sleep states. The machine-learning algorithm then determines the sleep state 60 of the test subject using a continuous measure of sleep depth determined from a weighted combination of the scores for each sleep state of the set of sleep states.

The method of determining S30 the sleep state 60 of the test subject may further comprise calculating one or more sleep metrics for the test subject. Sleep metrics that may be calculated include Total Sleep Time (TST), Wake after Sleep Onset, and REM Onset Latency (ROL). These metrics may be used to provide a summary view of sleep quality and duration within sleep diaries, or be used for the screening or diagnosis of sleep conditions.

In the present method, the at least one cardiorespiratory feature 8 for each of the training subjects (when training the machine learning algorithm) or the at least one cardiorespiratory feature 8 of the test subject (when determining the sleep state of a new test subject) is derived using a feature extractor 110 trained using reference cardiorespiratory signals 104 for each of a plurality of reference subjects. The feature extractor 110 comprises a neural network, for example of a type discussed above.

The reference cardiorespiratory signals 104 in respect of each reference subject are temporally divided into a plurality of epochs. These epochs are generally of equal length, typically 30 seconds, although this is not essential and other lengths of epoch may be used. Each epoch is labelled with one of a set of sleep states comprising wake, rapid-eye movement (REM) sleep, light non-rapid eye movement (NREM) sleep, or deep NREM sleep. The set of sleep states may further comprise intermediate NREM sleep.

The reference cardiorespiratory signals 104 comprise one or more of heart rate, respiratory rate, a pulse waveform, and a respiratory waveform.

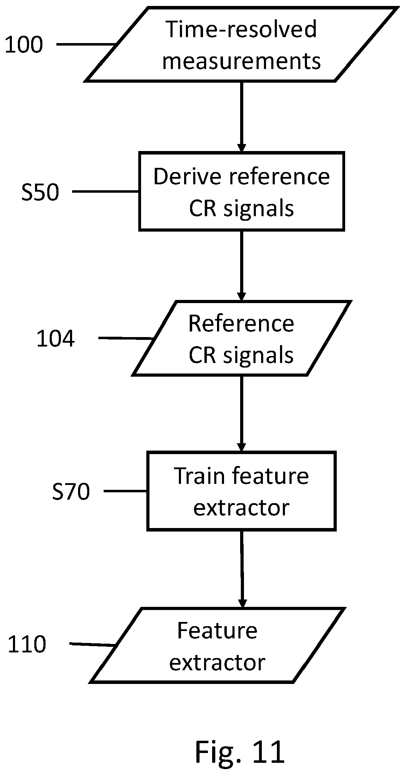

The flowchart of Fig. 11 shows a method of training the feature extractor 110 in order to illustrate the process by which the feature extractor 110 is obtained. These steps correspond to those on the left-hand side of Fig. 1 and are included as part of the methods of training the machine learning algorithm or of determining a sleep state 60 of a test subject that are discussed above in relation to Fig. 2, Fig. 9, and Fig. 10. The steps shown in Fig. 11 may be carried out at any suitable point prior to the steps of training S20 the machine-learning algorithm or determining S30 the sleep state 60. The steps shown in Fig. 11 produce the feature extractor 110 that is used to derive S6 the cardiorespiratory features 8. Alternatively, the steps shown in Fig. 11 may be carried out at an earlier time and not included in the methods above, in which case a feature extractor 110 pre-trained using the method of Fig. 11 is used to derive S6 the cardiorespiratory features in methods such as those shown in Fig. 2, Fig. 9, and Fig. 10.

The reference cardiorespiratory signals 104 are derived S50 from time-resolved measurements 100 from a plurality of sensors worn by the reference subjects. The plurality of sensors may include those typically used for sleep studies. An example is the SOMNOscreen system provided by SOMNOmedics Gmbh. To obtain the time-resolved measurements 100, the plurality of reference subjects wear the sensors and are given a sleep opportunity of several hours. Their sleep state is then monitored using the sensors. The plurality of sensors may include one or more of an accelerometer, a heart rate monitor, and a respiratory inductance pneumography (RIP) belt. An RIP belt measures expansion of the chest by a change in inductance of a belt comprising wire coils and placed around the chest. More than one RIP belt may be used. For example, two belts may be used with one placed on the thorax and the other on the abdomen.

As shown in Fig. 11, the method comprises deriving S50 the reference cardiorespiratory signals 104 from the time-resolved measurements 100.

Deriving the reference cardiorespiratory signals 104 may comprise deriving at least heart rate (HR) and respiratory rate (RR) 4 from the time-resolved measurements 100.

Heart rate (HR, also referred to as pulse rate) is obtained from conventional processing of the signal from the heart rate monitor, for example an electrocardiogram (ECG) sensor or an optical photo-plethysmography (PPG) sensor. This processing may comprise using a peak detection algorithm to detect each heart beat. Heart rate can be calculated from the inverse of the inter-beat interval.

The respiratory rate (RR, also referred to as breathing rate or respiration rate) is determined using measurements of the movement of the chest from the plurality of sensors. For example, these may be signals determined from the abdominal and/or thoracic RIP belts where these are used. Any other suitable sensor can be used that is able to provide a signal representative of movements or changes in another physiological parameter associated with the respiratory rate.

To determine the respiratory rate from the signals, a breath detection algorithm may be used to segment individual breath cycles in each signal, and may also be used to assess the quality of each breath waveform. Suitable methods of processing have been reported previously in [3-5].

The signal may be pre-processed to remove baseline noise. The breath detection algorithm may then use a moving average to smooth the signal over a suitable time window. Breath cycles can then be detected by identifying intercepts of the moving average signal with the inspiration and expiration branches of the signal. Peaks and troughs of the signal are defined, respectively, as the maximum and minimum between pairs of alternating inspiration and expiration intercepts.

Estimates of breath-by-breath RR can be derived as the inverse of the inter-breath interval. To reduce the risk of spurious measurements, the estimates may be averaged over a time window to produce a moving average of the RR to use in the reference cardiorespiratory signals 104. For example, the median breath-by-breath RR over a 30 second window may be used. Other averages or lengths of time window may be used, as appropriate for the particular situation. Estimates may be reported at a rate of 1 Hz, but other rates could be used.

Where RIP belts are used, the signal from one belt is sufficient to produce an estimate of RR. However, combining signals from both belts may help to improve signal quality. For example, the RR may be reported as the window-based averages between the RR estimates derived separately from the two RIP belts. To ensure signal consistency, estimates likely to be of low quality may be disregarded. For example, where the RR estimates from the RIP belts diverge by more than a predetermined amount (e.g. 3 breaths/min), no RR estimates may be reported for that time window.

Further steps may be applied to smooth the RR associated with each epoch in the reference cardiorespiratory signals 104. For each epoch, the RR input feature may be calculated as the mean RR in a time window (e.g. a 10 minute window) centred on that epoch.

The signal used to determine the respiratory rate (e.g. from the RIP belts) may also be used to determine a signal quality index (SQI) of the time-resolved measurements 100. Once breath cycles have been identified, the quality of each breath cycle can be assessed, for example using a morphological SQI algorithm. An example of a suitable algorithm applied to arterial blood pressure pulses is given in [5]. For each epoch, the SQI may be calculated similarly as the RR for the epoch, i.e. as the mean SQI in a time window (for example of 10 minutes) centred on that epoch. The SQI may be calculated concurrently with the RR, and may be used to exclude low quality breath cycles from contributing to the estimate of RR in the reference cardiorespiratory signals 104. For example, any breath cycles having an SQI below a threshold may be excluded from contributing to the moving average of the RR.

The reference cardiorespiratory signals 104 may further comprise a pulse waveform and/or a respiratory waveform. In the case of the reference cardiorespiratory signals 104, the pulse waveform and respiratory waveform are derived from the time-resolved measurements 100.

The pulse waveform is a time-resolved waveform representative of the subject's pulse or cardiac activity, and may be used to derive the heart rate. The pulse waveform may be representative of the amplitude of the signal from the heart rate monitor. The pulse waveform will typically have higher time resolution than the heart rate.

For example, the pulse waveform may be obtained from the ECG or PPG signals in a typical PSG study. The pulse waveform may be produced by applying signal processing to the ECG signal to smooth and/or filter the ECG signal.

The respiratory waveform is a time-resolved waveform representative of the subject's breathing, and may be used to derive the respiratory rate. The respiratory waveform may be representative of the amplitude of movement of the subject's chest as measured by the plurality of sensors, for example being representative of the amplitude of the signal from abdominal and/or thoracic RIP belts where these are used. The respiratory waveform may also be based on a respiratory effort signal derived from ECG and/or PPG measurements.

Deriving S50 the reference cardiorespiratory signals 104 from the time-resolved measurements 100 comprises down sampling and/or filtering the time-resolved measurements 100. Additional performance gains can be achieved using a "distribution matching" process, whereby signal processing steps are applied to the time resolved measurements 100 when deriving S50 the reference cardiorespiratory signals 104 to increase the similarity between the properties of the reference cardiorespiratory signals 104 and the corresponding properties of the training cardiorespiratory signals 4 and input cardiorespiratory signals 5 that will be derived S2 from the video images 2. This can include properties such as their frequency content.

For example, from the high frequency ECG (>100Hz) typically used in PSG studies, it is possible to calculate a high-resolution instantaneous heart rate (as previously used for sleep stage classification by Sridhar et al. [7]). However, it is not feasible to derive such a high-frequency heart rate using signals derived from a 20 FPS video camera. Applying additional signal processing steps to the time resolved measurements such that the signal properties of the reference cardiorespiratory signals 104 are more similar to the signal properties of the input or training cardiorespiratory signals 4, 5 avoids the possibility of the feature extractor learning to use characteristics in the source dataset of time-resolved measurements 100 which are not available in the target dataset of video images 2 during transfer learning. This mitigates the problem commonly referred to as distributional shift. For example, the time-resolved measurements 100 may be down-sampled to the same or approximately the same sampling rate as that of the video images 2. This means the sampling rate of the reference cardiorespiratory signals 104 will match that of the training cardiorespiratory signals 4 and input cardiorespiratory signals 5.

For the pulse waveform, an example of a suitable combination of processing of the ECG signal to arrive at the pulse waveform is the Pan-Tompkins algorithm, followed by a Butterworth bandpass filter (for example with a passband of 40-168 Hz, and Gaussian smoothing. This results in a pulse waveform that is morphologically similar to the pulse waveform derived from the video images for the training cardiorespiratory signals 4 and input cardiorespiratory signals 5.

The method comprises training S70 the feature extractor 110 using the reference cardiorespiratory signals 104. As shown on the left-hand side of Fig. 1, the feature extractor 110 is trained S70 by combining the feature extractor 110 with a reference classifier 112 to form a transfer network 114. The reference CR signals 104 derived S50 from the time-resolved measurements 100 from the sensors worn by the reference subjects are then fed into the transfer network 114. Within the transfer network 114, the CR features 8 from the penultimate layer of the feature extractor 110 are fed to the reference classifier 112. The transfer network 114 is trained to determine a sleep state 62 for each of the reference subjects from the reference CR signals 104. The training may comprise end-to-end training using back propagation. The transfer network 114 may be trained to minimise a loss function, for example a cross-entropy loss:

$$-\sum_{c=1}^{M} y_{o,c} \log(p_{o,c}) \qquad (1)$$

where M is the number of classes (which will be 5 when using the set of sleep states including intermediate NREM sleep), $y_{o,c}$ is a binary indicator of the true class and $p_{o,c}$ is the model output probability for class c. In the examples discussed below, the Adam optimiser was used with standard hyper-parameters and the transfer network 114 trained for a maximum of 10 epochs, with early stopping when the validation loss increases. A linear classifier was used for the reference classifier 112.

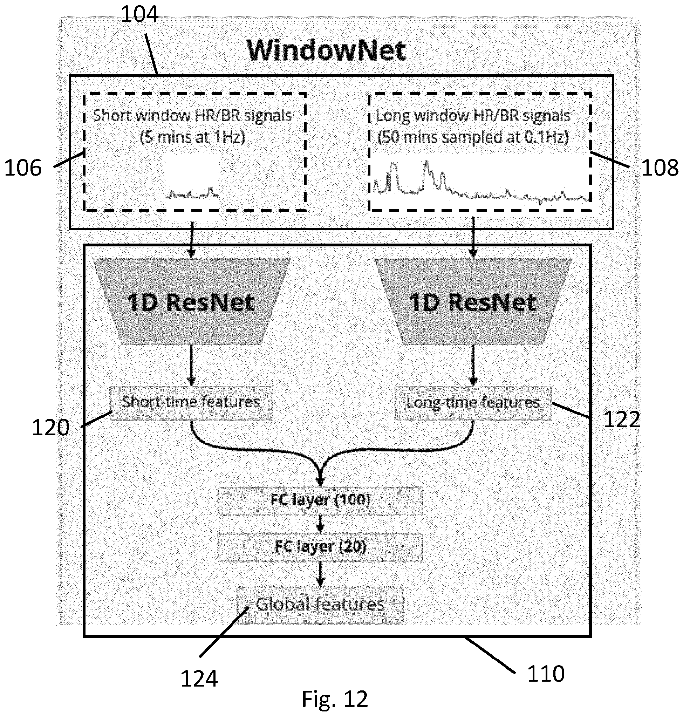

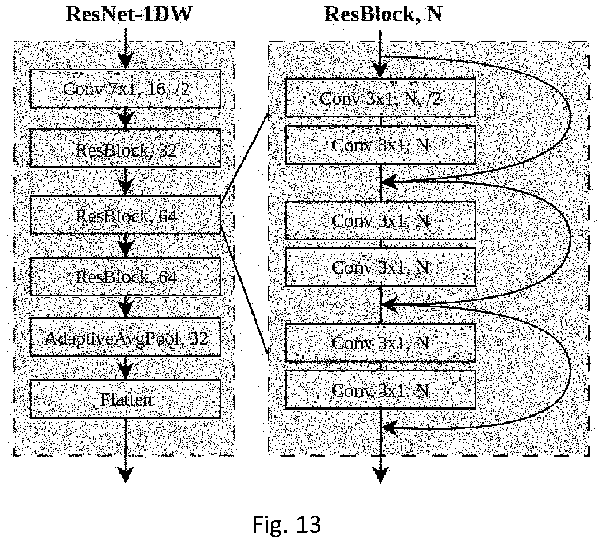

The architecture of the feature extractor 110 where the reference cardiorespiratory signals 104 comprise HR and RR is further illustrated in Fig. 12 and Fig. 13. This architecture is referred to as "WindowNet" and is used to derive the results in Experiment 1 below. This architecture is merely one example of a suitable architecture of the feature extractor 110, and other architectures may be used as appropriate.

A signal quality index (SQI) may be used to improve performance when training S70 the feature extractor 110. For example, low quality sections of the reference cardiorespiratory signals 104 (as indicated by the SQI being below a predetermined threshold) may be set to zero before the reference cardiorespiratory signals 104 are used to train S70 the feature extractor 110. Periods of low quality are strongly correlated with wakefulness, since they are often induced by motion artefacts. By zeroing out these sections, but not excluding them from the reference cardiorespiratory signal 104 entirely, the feature extractor 110 can still learn which periods are of low quality, whilst preventing the feature extractor 110 from overfitting to signal noise. An example of a suitable SQI for the heart rate signal is as follows

$$\text{SQI}_t^{\text{ECG}} = \begin{cases} 1 & \text{if } |\text{HR}_t^{\text{ECG}} - \text{HR}_t^{\text{Ox}}| \leq 3 \\ 0, & \text{otherwise} \end{cases} \qquad (2)$$

This sets the SQI to 1 when there is good agreement between the heart rates derived from ECG $HR_t^{ECG}$ and pulse oximetry $HR_t^{Ox}$. Otherwise, the SQI is zero. Periods in which the SQI is zero are considered low quality periods, and the heart rate is set to zero. A corresponding SQI can be derived for the respiratory rate signal using agreement between the breathing rates determined from the abdomen and thorax RIP belts.

The reference cardiorespiratory signals 104 comprise a first plurality of reference cardiorespiratory signals 106 and a second plurality of reference cardiorespiratory signals 108. The feature extractor 110 therefore uses two input streams, one from each plurality of reference cardiorespiratory signals.

The first and second pluralities of reference cardiorespiratory signals 106, 108 differ in one or more signal characteristics. In particular, the signal characteristics comprise sampling rate and duration. Duration here refers to the duration of a window around each epoch that is used to calculate the value of the signals for that epoch.

The first plurality of reference cardiorespiratory signals 106 may have a sampling rate that is at least 2 times greater than that of the second plurality of reference cardiorespiratory signals 108, optionally at least 5 times greater, optionally at least 10 times greater. The first plurality of reference cardiorespiratory signals 106 may have a duration that is at least 2 times less than that of the second plurality of reference cardiorespiratory signals 108, optionally at least 5 times less, optionally at least 10 times less. In the example of Fig. 12, the first plurality of reference cardiorespiratory signals 106 comprises 5-minute windows of stacked HR/BR data sampled at 1 Hz. The second plurality 108 of reference cardiorespiratory signals comprises 50-minute windows sampled at 0.1 Hz. In both cases, the windows are centred around the target epoch for classification. Of course, any other suitable values for the sampling rate and window lengths may be used as appropriate. The input streams in the form of the reference cardiorespiratory signals 104 thereby constitute short and long windows of heart rate (HR) and breathing rate (BR) time-series centred around the epoch for which a for sleep stage is to be determined.

Using the first and second pluralities of reference cardiorespiratory signals 106, 108, the feature extractor 110 extracts a first plurality of features 120 from the first plurality of reference cardiorespiratory signals 106 and a second plurality of features 122 from the second plurality of reference cardiorespiratory signals 108. In Fig. 12, each window of data around the epoch for which a sleep state is to be determined is passed through a 1D "ResNet" model [2], which transforms the inputs into the respective pluralities of features over short and long timescales. This enables the feature extractor 110 to utilise both short- and long-term information in the input vital sign data. The detailed structure of the ResNet model used to derive each of the first and second pluralities of features 120, 122 is shown in Fig. 13.

The feature extractor 110 combines the first and second pluralities of features 120, 122 into a plurality of output features 124 (also referred to as global features). The combination can involve not merely concatenating the features, but also weighting the first and second pluralities of features 120, 122 and reducing the number of features. The combination may be achieved with any suitable algorithm, such as a multilayer perceptron. The plurality of output features 124 is preferably smaller than a total number of features in the first and second pluralities of features 120, 122. In Fig. 12, the first and second pluralities of features 120, 122 are passed through fully connected (FC) layers, which produce the plurality of output features 124. During training S70 of the feature extractor 110, the output features 124 will then be passed into a linear classification layer to produce a sleep stage classification.

Since the feature extractor 110 uses two inputs over different timescales, these should also be provided when the feature extractor 110 is transferred for use in the method of training or of determining a sleep state of a test subject. Therefore, the training cardiorespiratory signals 4 comprise a first plurality of training cardiorespiratory signals and a second plurality of training cardiorespiratory signals. The first and second pluralities of training cardiorespiratory signals differ in one or more signal characteristics, such as sampling rate and duration, just as for the reference cardiorespiratory signals. The signal characteristics of the first and second pluralities of training cardiorespiratory signals should match the signal characteristics of the first and second pluralities of reference cardiorespiratory signals 106, 108. Similarly, the input cardiorespiratory signals 5 comprise a first plurality of input cardiorespiratory signals 5 and a second plurality of input cardiorespiratory signals. The first and second pluralities of input cardiorespiratory signals 5 differ in one or more signal characteristics, such as sampling rate and duration. The signal characteristics of the first and second pluralities of input cardiorespiratory signals should match the signal characteristics of the first and second pluralities of reference cardiorespiratory signals 106, 108.

The method is not limited to using only two pluralities of cardiorespiratory signals, and in general further pluralities of signals with different characteristics may be used to derive further pluralities of features.

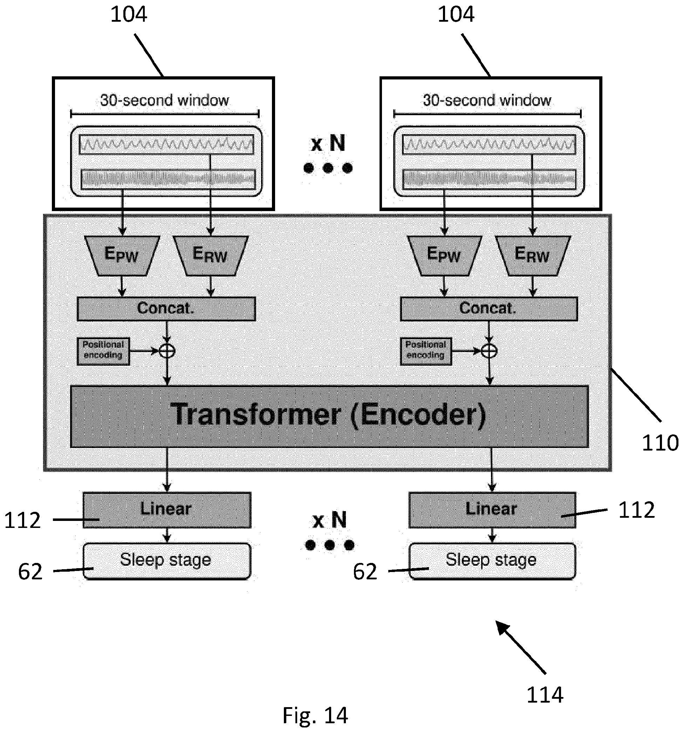

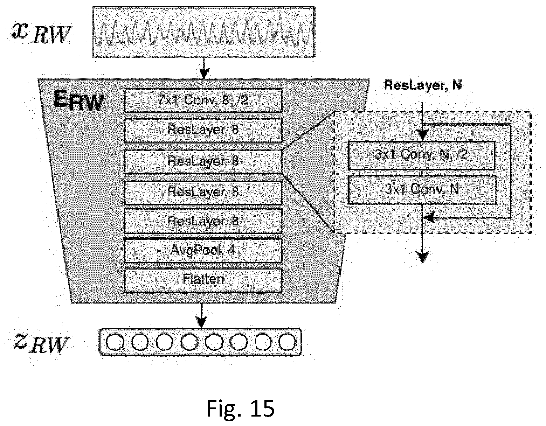

An alternative architecture of the feature extractor 110 where the reference cardiorespiratory signals 104 comprise pulse waveforms and respiratory waveforms is illustrated in Fig. 14 and Fig. 15. This architecture is referred to as "SleepVST" and is used to derive the results in Experiment 2 below. In Fig. 14, the feature extractor 110 is shown in the context of its training using the reference cardiorespiratory signals 104 as part of the transfer network 114.

Each 30-second window of pulse waveforms and respiratory waveforms making up the reference cardiorespiratory signals 104 is passed to an encoder. The encodings are then concatenated and passed to a transformer. Finally, a linear layer acting as the reference classifier 112 turns the transformer outputs into sleep states 62.

In this implementation, patchification is used and the pulse waveforms and respiratory waveforms are divided into N windows, i.e. patches, drawing on ideas from PatchTST [12]. Non-overlapping 30-second windows are used, since this is the interval at which sleep stages are typically annotated according to AASM guidelines. The patchified inputs for the pulse waveform and respiratory waveform are denoted as $x_{PW} \in \mathbb{R}^{300 \times N}$ and $x_{RW} \in \mathbb{R}^{150 \times N}$ respectively, where N is the sequence length.

Within each window, the patchified time series channels are passed to separate encoding layers $E_{PW}$ and $E_{RW}$, which transform them into feature vectors, $z_{PW} \in \mathbb{R}^{D_{PW} \times N}$ and $z_{RW} \in \mathbb{R}^{D_{RW} \times N}$. The feature vectors for each window are then concatenated, producing a sequence of input feature vectors $z_i \in \mathbb{R}^{(D_{PW} + D_{RW}) \times N}$ which are passed to a transformer encoder.

Identical, shallow 1D ResNet models are used for $E_{PW}$ and $E_{RW}$. This is illustrated in Fig. 15. A convolutional encoder design compresses the waveform patches into lower-dimensional feature vectors. This allows $D_{PW}$ and $D_{RW}$ to be kept small, reducing the computational and memory complexity of the downstream transformer and the likelihood of over-fitting particularly when transferring the feature extractor 110 to the smaller video dataset.

For the transformer layer, a transformer encoder model is used with sinusoidal position encodings to turn the sequence of input features $z_i$ into an output sequence of features $z_o$ with an identical feature dimension.

The attention mechanism of the transformer means that, at each intermediate layer, each output sequence element can be influenced by every other element in the sequence in an input-dependent way. This inductive bias makes the transformer a highly effective architecture for time-series problems.

For the transfer network 114, a linear classification layer $W_C \in \mathbb{R}^{C \times (D_{PW} + D_{RW})}$ is used as the reference classifier 112 to transform the sequence of output feature vectors $z_o$ into a sequence of sleep stage probabilities $y \in \mathbb{R}^{C \times N}$, where the number of classes C corresponds to the number of sleep stages (i.e. N1, N2 etc.) and depends on the sleep classification strategy used.

When the feature extractor 110 is transferred for use in the methods of training a machine learning network or determining a sleep state of a test subject, a small number of the network parameters may be adjusted to account for the differences in the data characteristics between the time-resolved measurements 100 and the video images 2. Training S70 the feature extractor may therefore comprise adjusting one or more parameters of the feature extractor 110 after training the transfer network 114.

Figs. 1, 2, and 9 to 11 illustrate methods including steps performed in a computer apparatus 200. In these drawings, the steps of the method are performed in functional blocks of the computer apparatus 200 (shown as rectangles). The functional blocks process data (shown as parallelograms) representing the various information described in detail above.

The computer apparatus 200 may be implemented as a computer apparatus executing a computer program. In this case, the computer program is capable of execution by the computer apparatus and is configured, on execution, to cause the computer apparatus to perform the method including the steps of the functional blocks. Such a computer apparatus may be any type of computer system but is typically of conventional construction. The computer program may be written in any suitable programming language.

The computer program may be stored on a computer-readable storage medium, which may be of any type, for example: a recording medium which is insertable into a drive of the computing system and which may store information magnetically, optically or opto-magnetically; a fixed recording medium of the computer system such as a hard drive; or a computer memory.

## Results

*Experiment 1*

Experiments were carried out using vital-sign estimates comprising heart rate (HR) and breathing rate (BR) time series estimated from the Sleep Heart Health Study (SHHS) dataset. SHHS is a public-access dataset of polysomnographic studies including the recording of 5247 polysomnograms (PSGs), usually at participants' homes, which have been scored according to the AASM standard by a sleep physiologist. The PSGs have a standard equipment montage including EEG, EMG, ECG and RIP signals, so ECG-based HR features and RIP-based RR were available.

A transfer network 114 was constructed as described above to classify annotated sleep stages available for 30-second epochs according to the AASM standard. The transfer network 114 was trained and validated using a subset of 3005 recordings which have higher quality vital-sign estimates. Quality is assessed using Hartigan's dip test for unimodality on the vital-sign estimates over the course of a recording. This removes recordings with multimodal vital sign distributions which is common when there are consistent artefacts in the estimates. The architecture of the feature extractor 110 is illustrated in Fig. 12 and Fig. 13, and was combined with a linear classifier to form the transfer network 114. The transfer network 114 was trained to minimise the cross-entropy loss using the Adam optimiser as described above.

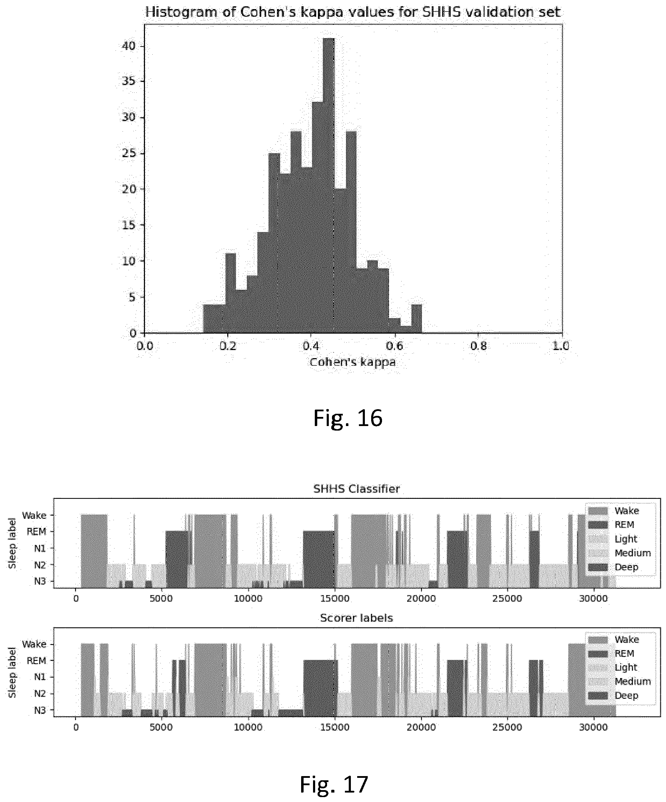

After training on the SHHS dataset, the distribution of Cohen's kappas for recordings from the validation set is plotted in Fig. 16. An example hypnogram derived from the output of the transfer network 114 (using an argmax over its class probabilities) is given in Fig. 17 (top panel). Sleep stage annotations from a sleep physiologist are also plotted (bottom panel). This example corresponds to the median accuracy in terms of the Cohen's kappa statistic ($\kappa \approx 0.4$). The transfer network has correctly identified the four REM cycles and extended periods of wakefulness.

Following training of the feature extractor 110 on the SHHS dataset, the trained feature extractor 110 was transferred for use on a smaller, new dataset referred to as the Oxford Sleep Volunteers (OSV) dataset. This was derived from 50 healthy participants, and comprised 50 overnight recordings of pulse rate, RR, and movement measures (derived using a proprietary algorithm, Oxehealth Ltd.).

The feature extractor was used to derive cardiorespiratory parameters from the OSV dataset, which were used along with the measures of movement to train another machine learning algorithm, according to the method of Fig. 2 and as illustrated on the right-hand side of Fig. 1. The machine-learning algorithm used was a random forest classifier. The 10 measures of subject movement 6 discussed above were used. The feature extractor 110 was constructed to produce 10 cardiorespiratory features. The number of features was kept small to prevent over-fitting.

The machine-learning algorithm was trained and evaluated using k-fold cross-validation. With k set to 10, 10 non-overlapping folds of 4-5 recordings as used, training the machine-learning algorithm on 9 folds and testing on the remaining fold for the 10 possible permutations. An argmax over the output class probabilities of the random forest was used as the sleep stage classification. To handle interscorer variability, the machine-learning algorithm was trained only in epochs for which the agreement between human scorers evaluated exceeds a given threshold, in this case where two human scorers agree on the applicable sleep stage label.

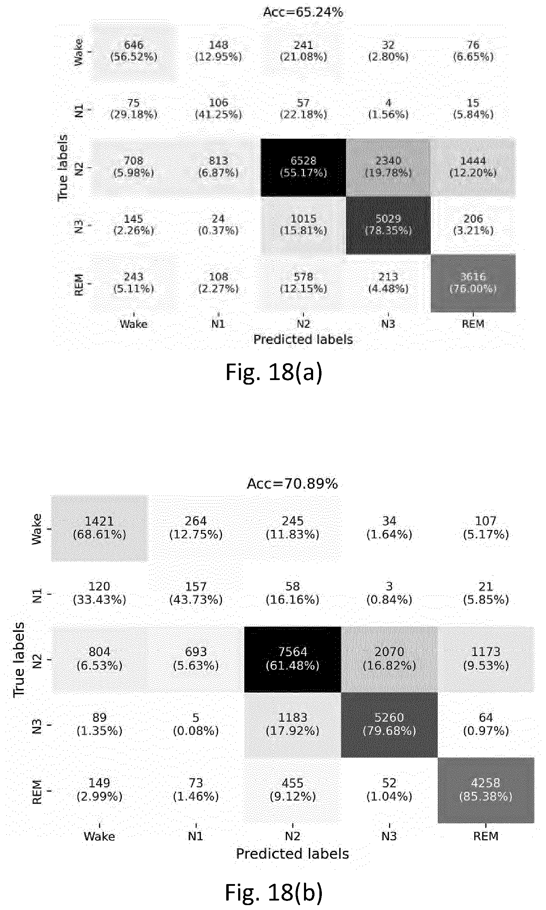

Confusion matrices for five class sleep staging are shown in Fig. 18. Fig. 18(a) shows the confusion matrix for a previous method that uses human-designed cardiorespiratory features (also referred to as a feature engineering approach). Fig. 18(b) shows the confusion matrix for the present transfer learning approach using deep cardiorespiratory features from the feature extractor. Using deep features results in an improvement of around 6% in 5-class sleep staging accuracy on epochs where two scorers agree.

Table 2 compares the accuracy of feature engineering and transfer learning approaches under different sleep stage categorisations using annotations from a single scorer. Accuracies are calculated by amalgamating the outputs of the five class model and the scorer labels. For example, when calculating accuracy on Wake-NREM-REM classification model classifications and scorer labels of either N1, N2, or N3 are converted to NREM.

**Table 2**

| Sleep-stage categorisation | Classification model accuracy (%) | |
| --- | --- | --- |
| | Feature engineering | Transfer learning |
| Sleep-Wake | **91.5** | 91.1 |
| Wake-NREM-REM | 79.3 | **83.1** |
| Wake-(N1+N2)-N3-REM | 63.0 | **68.5** |
| Wake-N1-N2-N3-REM | 57.9 | **64.3** |

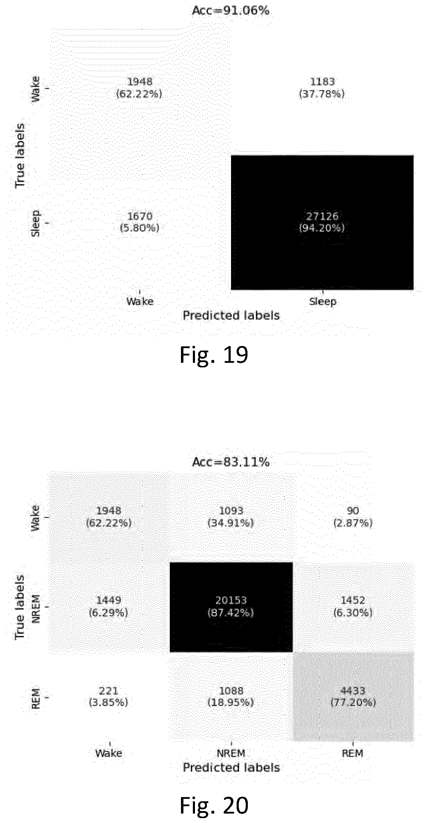

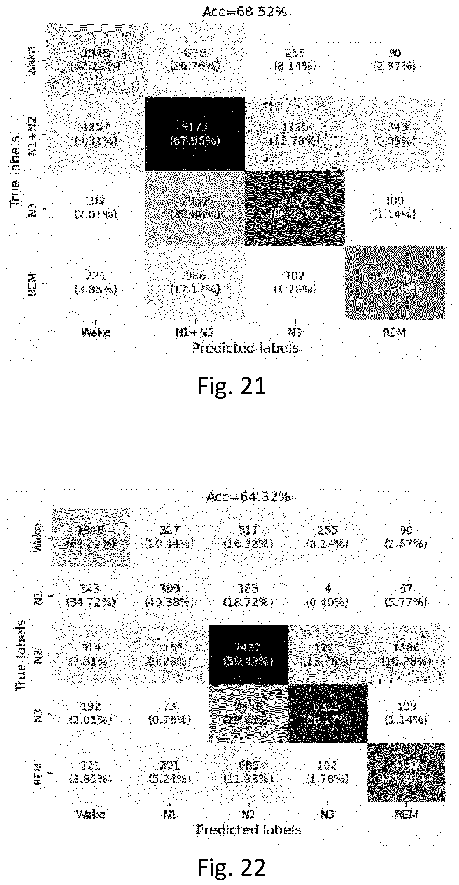

Confusion matrices using the transfer learning approach are given in Fig. 19 to Fig. 22. Fig. 19 shows the confusion matrix for Sleep/Wake categorisation. Fig. 20 shows the confusion matrix for Wake/NREM/REM categorisation. Fig. 21 shows the confusion matrix for Wake/N1+N2/N3/REM. Fig. 22 shows the confusion matrix for Wake/N1/N2/N3/REM, i.e. the AASM standard. The transfer learning model achieves a sensitivity of 94.2% and a specificity of 62.2% in classifying sleep.

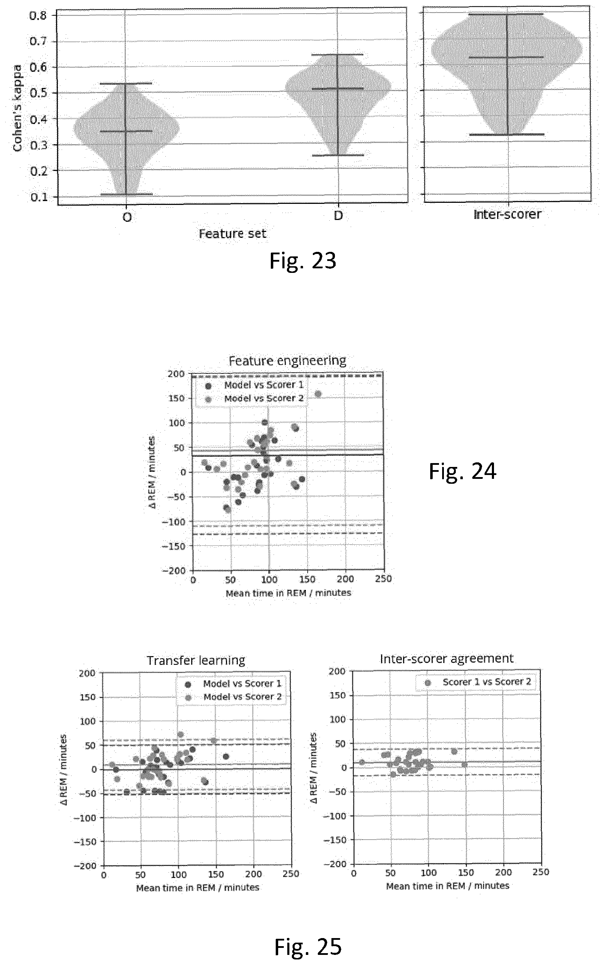

The Violin plots of Fig. 23 show the distribution of model-scorer Cohen's kappa statistics on recordings from the OSV dataset using feature engineering (O) and transfer learning (D) in the left-hand panel. The machine learning algorithms are evaluated against sleep stage annotations from a single scorer to make a fairer comparison with the inter-scorer case. Inter-scorer Cohen's kappa distributions calculated using annotations from two scorers are also plotted (right-hand panel).

Fig. 24 and the left-hand panel of Fig. 25 show Bland-Altman analysis of model-scorer agreement of total REM time for recordings in the OSV dataset using cardiorespiratory features derived from feature engineering and from transfer learning respectively. The model using transfer-learnt deep cardiorespiratory features shows greater agreement with both human scorers than the feature engineering model. Scorer-scorer agreement is shown in the right-hand panel of Fig. 25 for comparison.

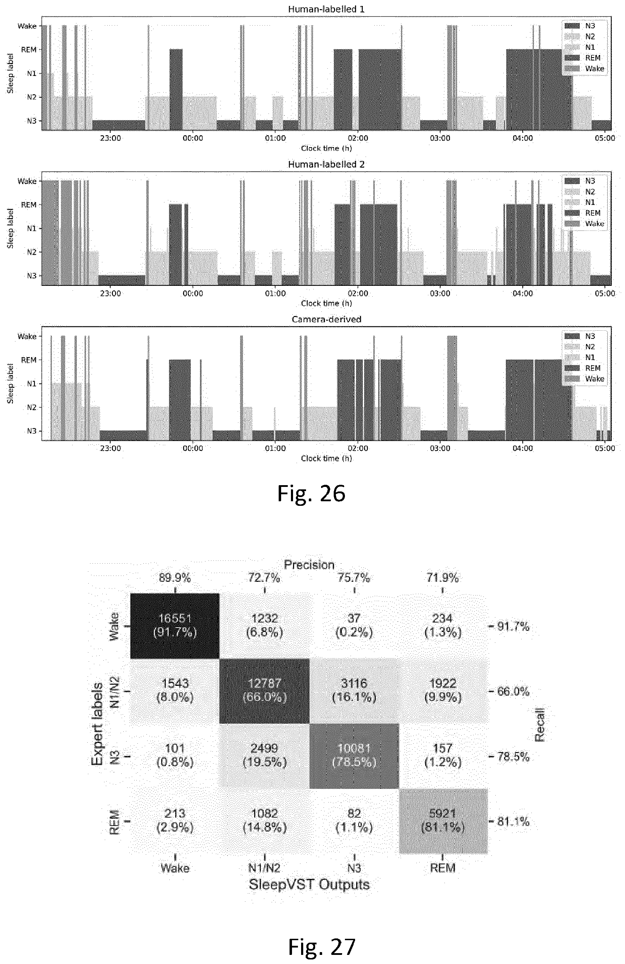

An example hypnogram produced by the machine-learning model trained using the transfer learnt cardiorespiratory features is shown in Fig. 26 (bottom panel) along with the annotations of two sleep physiologists (top and middle panels). The model has broadly identified the three main REM cycles in agreement with the two scorers.

From the results above, it is apparent that transfer learning provides an improvement over previous feature-engineering approaches. The average accuracy of non-contact sleep stage classification model was improved by around 6% by using transfer learning, when classifying sleep according to the AASM standard on epochs for which two physiologists agree. The violin plot of Fig. 23 shows that this equates to an improvement in per-subject accuracy across the dataset, with a large improvement to the lower quartile accuracy. This improvement in sleep stage classification accuracy also directly translates to an improvement in the accuracy of the estimate for total REM time observed in Fig. 24. The four-class classification accuracy of 68.5% reported in Table 2 is a large improvement over the 40.5% accuracy reported by previous camera-based sleep staging work such as that of Nochino et al. [8]. Sensitivity and specificity in binary sleep-wake classification (94.2%/62.2%) compares favourably with existing actigraphy methods. These have been reported to achieve 96.5% sensitivity and 32.9% specificity respectively, yet are still deemed an effective sleep monitoring technique for applications such as identifying circadian rhythm disorders. This indicates that the present method is already be accurate enough to provide benefit in a clinical setting.

*Experiment 2*

The specific implementation used in this experiment uses the Sleep Vital Signs Transformer, or SleepVST, discussed above in connection with Fig. 14 and Fig. 15.

A default sequence length of N = 240, i.e. two hours was used in the experiment. This was chosen to be slightly longer than a typical sleep cycle to enable the model to learn to identify this important physiological phenomena.

The model was trained to minimise the multi-class cross-entropy loss using the AdamW optimiser with default hyper-parameters. During training, batches were created by sampling sequences of length N with a step of 5 minutes from each recording. To improve training stability, pre-layer normalisation was employed within the transformer encoder layer.

Each training run was performed using a single NVIDIA A10 GPU with 24 GB RAM. Using a sequence length of two hours (N=240) and the default architecture, a batch size of 256 was used, the largest power of two that fitted on the GPU. Early-stopping was employed to terminate training once there had been no improvement in the validation loss for three consecutive epochs, restoring the model checkpoint that achieved the minimum value of validation loss.

After training, the model was applied to arbitrary length input sequences by reapplying it at regular step intervals.

Measures of subject movement were determined based on the measures $f_1$ to $f_4$ in Eq. (0.1) to Eq. (0.6) above for each of the three regions head (H), torso (B), and outer (O), for time windows $\Delta$ of 30 seconds and 300 seconds, and for thresholds $\delta$ of 0.01, 0.1, and 1. This gives 30 unique time-series features.

Additionally, timeshifted features $f_i(t + T)$ for T values of -90, 0, and +90 seconds were used. This gives a total of 90 motion features for each sleep epoch.

From the SHHS dataset, 500 participants were randomly selected who participated in both visits, to form a test set of 1000 nights of data. This approach ensured that no participant appeared in both the training/validation and test splits.

This experiment also used data from the multi-ethnic study of atherosclerosis (MESA) [9], another dataset of contact-sensor PSG recordings and sleep stages annotated according to AASM guidelines. From the MESA dataset, the same test set split of 204 nights was used as in Kotzen et al. [10].

The remaining nights from both the SHHS and MESA datasets were pooled together and randomly split into training and validation sets using a 75:25 split.

To transfer the SleepVST model to video, a similar approach was used as for Experiment 1. The classification layer learnt during pre-training on the contact sensor dataset is removed and the model is used as a feature extractor. The feature extractor is applied to sequences of cardiorespiratory waveforms measured from video. A new classification head is then trained that uses these features plus the motion features to classify individual sleep epochs.

This allows the representational capacity of the transformer model, trained on the larger contact-sensor datasets, to be leveraged whilst also enabling the incorporation of motion information on the much smaller video dataset.

Table 3 compares the performance of the SleepVST model with prior work on cardio-respiratory sleep staging on these datasets. These have used input modalities such as the photoplethysmogram (PPG) waveform and the ECG-derived heart rate. Using waveforms derived from the ECG and thoracic respiratory signals, as discussed above, Sleep-VST outperforms all prior methods for wearable cardio-respiratory sleep staging on both datasets. Sleep staging accuracy has commonly been reported using the mean Cohen's kappa over individual recordings $\kappa_\mu$ and/or using the Cohen's kappa calculated over all sleep-epoch classifications in the dataset combined $\kappa_T$. Both of these statistics are reported to make fairer comparisons with prior work.

**Table 3**

| Dataset | N | Method | Modalities | Cohen's $\kappa$ | | Accuracy / % | |
|---|---|---|---|---|---|---|---|
| | | | | $\kappa_\mu$ | $K/_T$ | Acc$_\mu$ | ACC$_T$ |
| SHHS | 296 | Bakker et al. [4] | HR+Thor*+Air† | - | 0.64 | - | 76.7 |
| | 800 | Sridhar et al. [52] | HR (ECG) | 0.65 | 0.66 | 77.3 | 77.0 |
| | 1000 | SleepVST | ECG+Thor | **0.73** | **0.75** | **82.8** | **83.0** |

(continued)

| Dataset | N | Method | Modalities | Cohen's $\kappa$ | | Accuracy / % | |
|---------|---|--------|-----------|------|------|------|------|
| | | | | $\kappa_\mu$ | $\kappa_T$ | Acc$_\mu$ | ACC$_T$ |
| MESA | 296 | Bakker et al. [4] | HR+Thor*+Air† | - | 0.68 | - | 79.8 |
| | 194 | Sridhar et al. [52] | HR (ECG) | - | 0.69 | - | 80.0 |
| | 204 | Kotzen et al. [26] | PPG | - | 0.73‡ | - | 82.6‡ |
| | 204 | SleepVST | ECG+Thor* | **0.76** | **0.77** | **85.1** | **85.2** |

\* Thoracic respiratory effort. †Nasal airflow. \* From reported confusion matrices.

The performance of the SleepVST model was then evaluated after transfer to the OSV video dataset. Training and evaluating on the 50 nights used 10-fold cross-validation with non-overlapping folds of 10 recordings.

Table 4 compares the results with prior video-based sleep staging work. The frame rate (FPS) and wavelength(s) of the camera(s) used in data collection are reported where available. SleepVST significantly outperforms prior sleep staging work in terms of both accuracy and the Cohen's κ statistic.

**Table 4**

| Population | N | Method | Modalities** | Cohen's $\kappa$ | | Accuracy / % | |
|-----------|---|--------|-------------|------|------|------|------|
| | | | | $\kappa_\mu$ | $\kappa_T$ | Acc$_\mu$ | ACC$_T$ |
| Infants Adults | 8 | Kamon et al. [25] | Motion | 0.26 | - | 48.0 | - |
| | 6 | Nochino et al. [38] | Motion | 0.19 | - | 40.5 | - |
| | 46 | van Meulen et al. [59] | HW* | 0.49 | 0.49‡ | 67.9 | 67.9* |
| | 50 | Carter et al. [7] | HR+BR+Motion | 0.61 | 0.64‡ | 73.4 | 74.3‡ |
| | 50 | SleepVST | HW*+BW†+Motion | **0.68** | **0.71** | **77.7** | **78.8** |

\*\*Camera-derived. \*Cardiac pulse (heart) waveforms. †Respiration (breathing) waveforms. \* From reported confusion matrices.

Fig. 27 shows the confusion matrix for four-class video based sleep staging between model and scorer outputs on the OSV dataset, summed over all sleep epochs. Particularly high accuracy is observed in distinguishing Wake and REM.

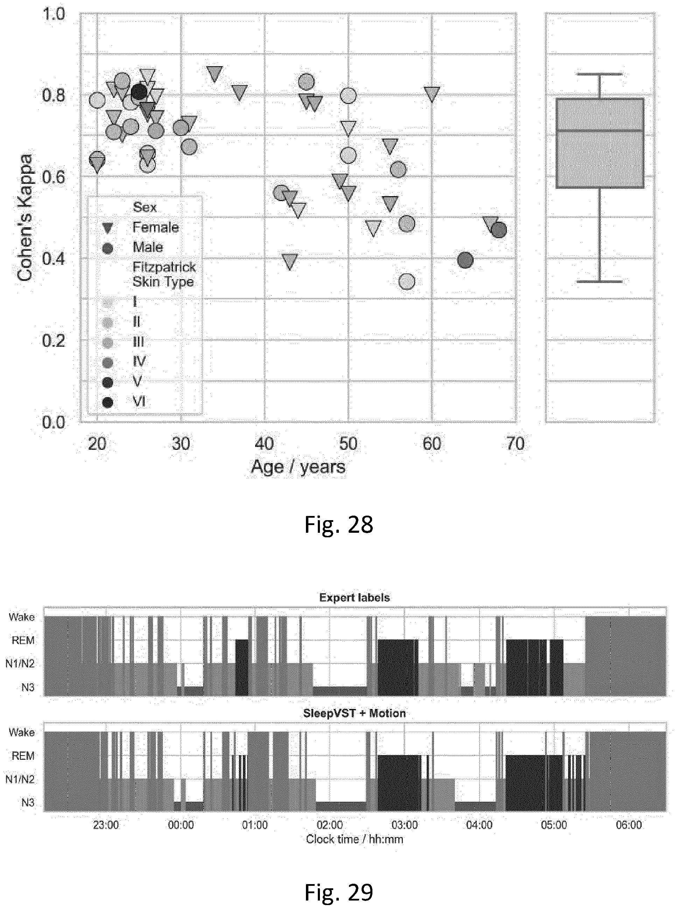

Fig. 28 shows the distribution of Cohen's κ values across participants in the OSV dataset as a function of age, sex, and Fitzpatrick skin type. A decrease in performance is observed with age, as observed in prior sleep staging work.

Fig. 29 shows an example sleep hypnogram generated from near-infrared video using the present method, along with an expert-annotated hypnogram for comparison. The model correctly identifies all three REM cycles, periods of deep sleep, and brief awakenings throughout the night.

Several ablation experiments were performed against simpler variants of the model.

Firstly, ablation studies were conducted with respect to the cardio-respiratory inputs. In Table 5, the performance of the SleepVST model is compared with simpler variants which use patches of heart rate $x_{HR} \in \mathbb{R}^{30 \times N}$ and/or breathing rate $x_{BR} \in \mathbb{R}^{30 \times N}$ time-series as inputs, instead of the underlying waveforms. When using the derived rates, linear projections are used for the input encoders because of the lower dimensionality of $x_{HR}$ and $x_{BR}$. Results are provided on both the contact sensor datasets, applying the SleepVST model directly after pre-training, and on the OSV video dataset after re-training the classification head with motion information.

**Table 5**

| Inputs | Cohen's $\kappa_T$ | | |
|--------|------|------|------|
| | SHHS | MESA | OSV |
| HR+BR | 0.683 | 0.722 | 0.681 |

(continued)

| | Cohen's $\kappa_T$ | | |
| --- | --- | --- | --- |
| Inputs | SHHS | MESA | OSV |
| HW+BR | 0.718 | 0.736 | 0.684 |
| 14R+BW | 0.723 | 0.758 | 0.702 |
| HW+BW | **0.749** | **0.765** | **0.708** |

Secondly, ablation studies were conducted with respect to the video data transfer strategy. In Table 6, the performance of three methods for transferring the SleepVST model to video data are compared with two alternative approaches:

1. Directly applying the SleepVST model to cardiorespiratory waveforms from video data.

2. Using SleepVST as a feature extractor and training the classification layer without using motion features.

3. Using SleepVST as a feature extractor and training the classification layer using motion features and cardio-respiratory features from SleepVST, i.e. the best-performing approach.

**Table 6**

| | $\kappa_\mu$ | $\kappa_T$ | $Acc_\mu$/% | $ACC_T$/% |
| --- | --- | --- | --- | --- |
| Direct apply | 0.510 | 0.536 | 66.7 | 67.3 |
| Transfer w/o motion | 0.660 | 0.689 | 76.4 | 77.4 |
| Transfer w/ motion | **0.677** | **0.708** | **77.7** | **78.8** |

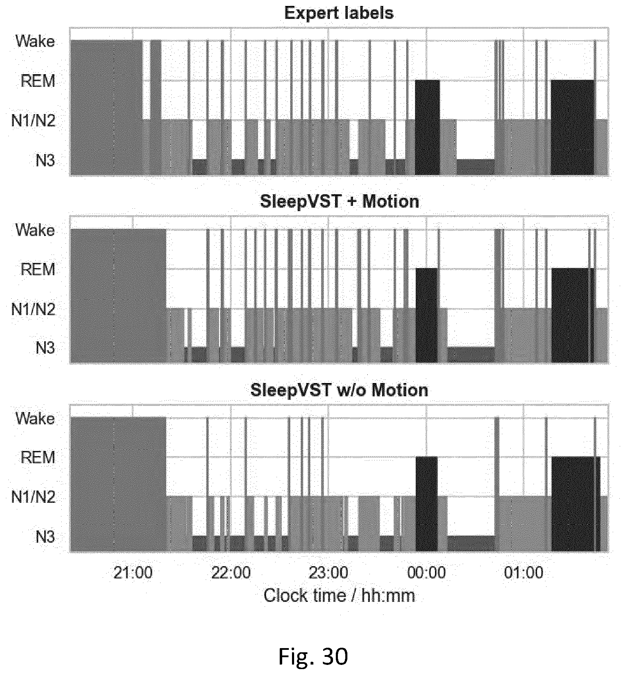

This demonstrates that training a new classifier for video data provides a significant improvement. The addition of motion features has a smaller impact on overall performance. However, it improves performance particularly in classifying periods of fragmented sleep. This is illustrated in Fig. 30, which shows example four-class hypnograms during fragmented sleep from the OSV dataset. The top panel shows expert-annotated labels. The middle panel shows labels from SleepVST with motion features. The bottom panel shows labels from SleepVST without motion features. The addition of motion improves the classification of short awakenings between 22.00 and 00.00.

Finally, ablation studies were conducted with respect to the motion features. Table 7 looks at the effectiveness of two main components of the motion feature set: the set of feature definitions $f_i$ and the set of regions R over which the features are calculated. An additional metric is defined and reported $\kappa_F$, which is the Cohen's $\kappa$ statistic computed

over epochs near transitions to/from Wake (W), i.e. where $\hat{y}_i^t \neq \hat{y}_i^{t+\Delta} = W$ or $\hat{y}_i^t = W \neq \hat{y}_i^{t+\Delta}$ for any epochs. This metric better quantifies accuracy during periods of sleep fragmentation such as in Fig. 30. It is observed that the addition of motion features is particularly advantageous for classification around Wake transitions. This is aided by the use of distinct regions R, and the use of features which measure gross movement within a time window ($f_1, f_2$) and those which measure elapsed time since major movements ($f_3, f_4$).

**Table 7**

| Ablation | Parameter Set | N[1] | Cohen's $\kappa$ | |
| --- | --- | --- | --- | --- |
| | | | $\kappa_T$ | $\kappa_F$ |
| Feature set | $f_i \in \{\}$ | 0 | 0.689 | 0.397 |
| | $f_i \in \{f_1, f_2\}$ | 36 | 0.697 | 0.467 |
| | $f_i \in \{f_3, f_4\}$ | 54 | 0.704 | 0.465 |
| | $f_i \in \{f_1, f_2, f_3, f_4\}$ | 90 | **0.708** | **0.491** |
| Region(s) | $R \in \{H \cup B\}$ | 30 | 0.702 | 0.461 |
| | $R \in \{H \cup B, O\}$ | 60 | 0.704 | 0.468 |
| | $R \in \{H, B, O\}$ | 90 | **0.708** | **0.491** |

[1]No. features.

**References**

[1] Yosinski J, Clune J, Bengio Y, and Lipson H. How transferable are features in deep neural networks? 2014 Nov 6. arXiv: 1411.1792.

[2] He K, Zhang X, Ren S, and Sun J. Deep Residual Learning for Image Recognition. Proceedings of the IEEE Conference on Computer Vision and Pattern Recognition. 2016: 770-8

[3] Jorge, J., Villarroel, M., Tomlinson, H. et al. Non-contact physiological monitoring of post-operative patients in the intensive care unit. npj Digit. Med. 5, 4 (2022). https://doi.org/10.1038/s41746-021-00543-z

[4] Villarroel, M., Jorge, J., Meredith, D. et al. Non-contact vital-sign monitoring of patients undergoing haemodialysis treatment. Sci Rep 10, 18529 (2020). https://doi.org/10.1038/s41598-020-75152-z

[5] W. Zong, T. Heldt, G. B. Moody and R. G. Mark, "An open-source algorithm to detect onset of arterial blood pressure pulses," Computers in Cardiology, 2003, 2003, pp. 259-262.

[6] Radha M, Fonseca P, Moreau A, Ross M, Cerny A, Anderer P, Long X, and Aarts R M. A deep transfer learning approach for wearable sleep stage classification with photoplethysmography, npj Digital Medicine 2021 Sep 15; 4. Number: 1.

[7] SridharN, Shoeb A, Stephens P, Kharbouch A, Shimol D B, Burkart J, Ghoreyshi A, and Myers L. Deep learning for automated sleep staging using instantaneous heart rate. npj Digital Medicine. 2020 Aug 20; 3. Number: 1.

[8] Nochino T, Ohno Y, Kato T, Taniike M, and Okada S. Sleep stage estimation method using camera for home use. Biomedical Engineering Letters 2019 Apr 24; 9:257-65

[9] Xiaoli Chen, Rui Wang, Phyllis Zee, Pamela L. Lutsey, Sogol Javaheri, Carmela Alcántara, Chandra L. Jackson, Michelle A. Williams, and Susan Redline. Racial/Ethnic Differences in Sleep Disturbances: The Multi-Ethnic Study of Atherosclerosis (MESA). Sleep, 38(6): 877-888, 2015.

[10] Kevin Kotzen, Peter H. Charlton, Sharon Salabi, Lea Amar, Amir Landesberg, and Joachim A. Behar. Sleep PPG-Net: A Deep Learning Algorithm for Robust Sleep Staging From Continuous Photoplethysmography. IEEE Journal of Biomedical and Health Informatics, 27(2):924-932, 2023. Conference Name: IEEE Journal of Biomedical and Health Informatics.

[11] Jessie P. Bakker, Marco Ross, Ray Vasko, Andreas Cerny, Pedro Fonseca, Jeff Jasko, Edmund Shaw, David P. White, and Peter Anderer. Estimating sleep stages using cardiorespiratory signals: validation of a novel algorithm across a wide range of sleep-disordered breathing severity. Journal of Clinical Sleep Medicine, 17(7):1343-1354, 2021.

[12] Yuqi Nie, Nam H.Nguyen, Phanwadee Sinthong, and Jayant Kalagnanam. A Time Series is Worth 64Words: Long-term Forecasting with Transformers. 2022.

The invention may be further described by the following numbered clauses:

1. A method comprising determining a sleep state of a test subject using input data from the test subject, wherein: the input data comprise: at least one measure of test subject movement; and at least one cardiorespiratory feature of the test subject; the input data are derived from video images of the test subject; and the at least one cardiorespiratory feature of the test subject is derived using a feature extractor trained using reference cardiorespiratory signals for each of a plurality of reference subjects, the feature extractor comprising a neural network, and the reference cardiorespiratory signals derived from time-resolved measurements from a plurality of sensors worn by the reference subjects.

2. A method according to clause 1, further comprising training the feature extractor using the reference cardiorespiratory signals.

3. A method according to clause 2, wherein training the feature extractor comprises combining the feature extractor with a reference classifier to form a transfer network and training the transfer network to determine a sleep state for each of the reference subjects, optionally by training the transfer network to minimise a loss function, for example a cross-entropy loss.

4. A method according to any one of the preceding clauses, wherein the reference cardiorespiratory signals in respect of each reference subject are temporally divided into a plurality of epochs, each epoch being labelled with one of a set of sleep states comprising wake, rapid-eye movement (REM) sleep, light non-rapid eye movement (NREM) sleep, or deep NREM sleep, optionally wherein in the set of sleep states further comprises intermediate NREM sleep.

5. A method according to any one of the preceding clauses, wherein the reference cardiorespiratory signals comprise one or more of heart rate, respiratory rate, a pulse waveform, and a respiratory waveform.

6. A method according to any one of the preceding clauses, wherein the reference cardiorespiratory signals comprise a first plurality of reference cardiorespiratory signals and a second plurality of reference cardiorespiratory signals, wherein the first and second pluralities of reference cardiorespiratory signals differ in one or more signal characteristics, optionally wherein the signal characteristics comprise sampling rate and duration.

7. A method according to clause 6 wherein the feature extractor extracts a first plurality of features from the first plurality of reference cardiorespiratory signals and a second plurality of features from the second plurality of reference cardiorespiratory signals.

8. A method according to clause 7, wherein the feature extractor combines the first and second pluralities of features into a plurality of output features, optionally wherein the plurality of output features is smaller than a total number of features in the first and second pluralities of features.

9. A method according to any one of the preceding clauses, wherein the method further comprises deriving the reference cardiorespiratory signals from the time-resolved measurements.

10. A method according to clause 9, wherein deriving the reference cardiorespiratory signals from the time-resolved measurements comprises down sampling and/or filtering the time-resolved measurements.

11. A method according to any one of the preceding clauses, further comprising deriving the at least one cardiorespiratory feature of the test subject using the feature extractor.

12. A method according to any one of the preceding clauses, wherein the at least one cardiorespiratory feature of the test subject is derived from input cardiorespiratory signals using the feature extractor, optionally wherein the input cardiorespiratory signals comprise one or more of heart rate, respiratory rate, a pulse waveform, and a respiratory waveform.

13. A method according to clause 12, wherein the input cardiorespiratory signals comprise a first plurality of input cardiorespiratory signals and a second plurality of input cardiorespiratory signals, wherein the first and second pluralities of input cardiorespiratory signals differ in one or more signal characteristics, optionally wherein the signal characteristics comprise sampling rate and duration.

14. A method according to clauses 12 or 13, wherein the input cardiorespiratory signals are derived from video images of the test subject, optionally wherein the method further comprises deriving the input cardiorespiratory signals from the video images of the test subjects.

15. A method according to any one of the preceding clauses, further comprising deriving the at least one measure of test subject movement from the video images of the test subject.

16. A method according to any one of the preceding clauses, wherein the at least one measure of test subject movement comprises one or more of a measure of subject movement in a head region of the subject, a measure of movement in a torso region of the subject, and a measure of movement in an outer region of the video image.

17. A method according any one of the preceding clauses, wherein determining the sleep state of the test subject comprises applying a machine-learning algorithm to the input data, wherein the machine-learning algorithm outputs a determination of the sleep state of the test subject on the basis of training data in respect of a plurality of training subjects; the training data are derived from video images of the training subjects; the training data comprise for each of the training subjects: at least one measure of subject movement; and at least one cardiorespiratory feature of the subject; and the at least one cardiorespiratory feature for each of the training subjects is derived using the feature extractor.

18. A method according to clause 17, wherein the machine learning algorithm is a regression algorithm, and the machine-learning algorithm determines the sleep state of the test subject using a continuous measure of sleep depth, optionally wherein the regression algorithm is a neural network.

19. A method according to clause 17, wherein: the training data in respect of each training subject are temporally divided into a plurality of epochs, each epoch being labelled with one of a set of sleep states; and the machine learning algorithm is a classification algorithm that classifies the sleep state of the test subject as one of the set of sleep states, optionally wherein the classification algorithm is one of a logistic regression, k-nearest neighbours, a random forest classifier, and a neural network.

20. A method according to clause 17, wherein: the training data in respect of each training subject are temporally divided into a plurality of epochs, each epoch being labelled with one of a set of sleep states; the machine learning algorithm is a classification algorithm, optionally one of a logistic regression, k-nearest neighbours, a random forest classifier, and a neural network; the classification algorithm generates scores for each sleep state of the set of sleep states, the scores representing a confidence of the sleep state of the test subject being that sleep state of the set of sleep states; and the machine-learning algorithm determines the sleep state of the test subject using a continuous measure of sleep depth, the continuous measure being determined from a weighted combination of the scores for each sleep state of the set of sleep states.

21. A method according to clause 19 or 20, wherein the set of sleep states comprises wake, rapid-eye movement (REM) sleep, light non-rapid eye movement (NREM) sleep, or deep NREM sleep, optionally wherein in the set of sleep states further comprises intermediate NREM sleep.

22. A method according to any one of clauses 17 to 21, wherein the at least one cardiorespiratory feature for each of the training subjects is derived from training cardiorespiratory signals using the feature extractor, optionally wherein the training cardiorespiratory signals comprise one or more of heart rate, respiratory rate, a pulse waveform, and a respiratory waveform.

23. A method according to clause 22, wherein the training cardiorespiratory signals comprise a first plurality of training cardiorespiratory signals and a second plurality of training cardiorespiratory signals, wherein the first and second pluralities of training cardiorespiratory signals differ in one or more signal characteristics, optionally wherein the signal characteristics comprise sampling rate and duration.

24. A method according to clause 22 or 23, wherein the training cardiorespiratory signals are derived from video images of the training subjects.

25. A method according to any one of clauses 17 to 24, further comprising training the machine-learning algorithm using the training data.

26. A method of training a machine-learning algorithm to determine a sleep state of a subject, wherein: the method uses training data in respect of a plurality of training subjects; the training data are derived from video images of the training subjects; the training data comprise for each of the training subjects: at least one measure of subject movement; and at least one cardiorespiratory feature of the subject; the at least one cardiorespiratory feature for each of the training subjects is derived using a feature extractor trained using reference cardiorespiratory signals for each of a plurality of reference subjects, the feature extractor comprising a neural network, and the reference cardiorespiratory signals derived from time-resolved measurements from a plurality of sensors worn by the reference subjects; and the method comprises training the machine learning algorithm using the training data.

27. A method according to clause 25 or 26, further comprising deriving the at least one cardiorespiratory feature for each of the training subjects from training cardiorespiratory signals using the feature extractor, optionally wherein the training cardiorespiratory signals comprise one or more of heart rate, respiratory rate, a pulse waveform, and a respiratory waveform.

28. A method according to any one of clauses 25 to 27, further comprising deriving the training cardiorespiratory signals from the video images of the training subjects.

29. A method according to any one of clauses 25 to 28, further comprising deriving the at least one measure of subject movement from the video images of the training subjects.

30. A method according to any one of clauses 25 to 29, wherein the at least one measure of test subject movement comprises one or more of a measure of subject movement in a head region of the subject, measure of movement in a torso region of the subject, and a measure of movement in an outer region of the video image.

31. A computer program comprising, or a computer-readable storage medium having stored thereon, instructions which, when carried out by a computer, cause the computer to carry out a method according to any one of the preceding clauses.

32. A computer apparatus configured to determine a sleep state of a test subject using input data from the test subject, wherein: the input data comprise: at least one measure of test subject movement; and at least one cardiorespiratory feature of the test subject; the input data are derived from video images of the test subject; and the at least one cardiorespiratory feature of the test subject is derived using a feature extractor trained using reference cardiorespiratory signals for each of a plurality of reference subjects, the feature extractor comprising a neural network, and the reference cardiorespiratory signals derived from time-resolved measurements from a plurality of sensors worn by the reference subjects.

33. A computer apparatus for training a machine-learning algorithm to determine a sleep state of a subject, the apparatus configured to train the machine learning algorithm using training data in respect of a plurality of training subjects, wherein: the training data are derived from video images of the training subjects; the training data comprise for each of the training subjects: at least one measure of subject movement; and at least one cardiorespiratory feature of the subject; and the at least one cardiorespiratory feature for each of the training subjects is derived using a feature extractor trained using reference cardiorespiratory signals for each of a plurality of reference subjects, the feature extractor comprising a neural network, and the reference cardiorespiratory signals derived from time-resolved measurements from a plurality of sensors worn by the reference subjects.

**Claims**

1. A method comprising determining a sleep state of a test subject using input data from the test subject, wherein:

   the input data comprise: at least one measure of test subject movement; and at least one cardiorespiratory feature of the test subject;
   the input data are derived from video images of the test subject; and
   the at least one cardiorespiratory feature of the test subject is derived using a feature extractor trained using reference cardiorespiratory signals for each of a plurality of reference subjects, the feature extractor comprising a neural network, and the reference cardiorespiratory signals derived from time-resolved measurements from a plurality of sensors worn by the reference subjects.

2. A method according to claim 1, further comprising training the feature extractor using the reference cardiorespiratory signals, wherein training the feature extractor comprises combining the feature extractor with a reference classifier to form a transfer network and training the transfer network to determine a sleep state for each of the reference subjects, optionally by training the transfer network to minimise a loss function, for example a cross-entropy loss.

3. A method according to claim 1 or 2, wherein one or both of:

   a) the reference cardiorespiratory signals in respect of each reference subject are temporally divided into a plurality of epochs, each epoch being labelled with one of a set of sleep states comprising wake, rapid-eye movement (REM) sleep, light non-rapid eye movement (NREM) sleep, or deep NREM sleep, optionally wherein in the set of sleep states further comprises intermediate NREM sleep; and
   b) the reference cardiorespiratory signals comprise one or more of heart rate, respiratory rate, a pulse waveform, and a respiratory waveform.

4. A method according to any one of the preceding claims, wherein the reference cardiorespiratory signals comprise a first plurality of reference cardiorespiratory signals and a second plurality of reference cardiorespiratory signals, wherein the first and second pluralities of reference cardiorespiratory signals differ in one or more signal characteristics, optionally wherein the signal characteristics comprise sampling rate and duration,

   optionally wherein the feature extractor extracts a first plurality of features from the first plurality of reference cardiorespiratory signals and a second plurality of features from the second plurality of reference cardiorespiratory signals,
   further optionally wherein the feature extractor combines the first and second pluralities of features into a plurality of output features, optionally wherein the plurality of output features is smaller than a total number of features in the first and second pluralities of features.

5. A method according to any one of the preceding claims, wherein the method further comprises deriving the reference cardiorespiratory signals from the time-resolved measurements,
   optionally wherein deriving the reference cardiorespiratory signals from the time-resolved measurements comprises down sampling and/or filtering the time-resolved measurements.

6. A method according to any one of the preceding claims, wherein the at least one cardiorespiratory feature of the test subject is derived from input cardiorespiratory signals using the feature extractor, optionally wherein the input cardiorespiratory signals comprise one or more of heart rate, respiratory rate, a pulse waveform, and a respiratory waveform,
   optionally wherein one or both of:

   a) the input cardiorespiratory signals comprise a first plurality of input cardiorespiratory signals and a second plurality of input cardiorespiratory signals, wherein the first and second pluralities of input cardiorespiratory signals differ in one or more signal characteristics, optionally wherein the signal characteristics comprise sampling rate and duration; and
   b) the input cardiorespiratory signals are derived from video images of the test subject, optionally wherein the method further comprises deriving the input cardiorespiratory signals from the video images of the test subjects.

7. A method according to any one of the preceding claims, further comprising one or both of:

   a) deriving the at least one measure of test subject movement from the video images of the test subject; and
   b) deriving the at least one cardiorespiratory feature of the test subject using the feature extractor.

8. A method according any one of the preceding claims, wherein determining the sleep state of the test subject comprises applying a machine-learning algorithm to the input data, wherein

   the machine-learning algorithm outputs a determination of the sleep state of the test subject on the basis of training data in respect of a plurality of training subjects;

the training data are derived from video images of the training subjects;

the training data comprise for each of the training subjects: at least one measure of subject movement; and at least one cardiorespiratory feature of the subject; and

the at least one cardiorespiratory feature for each of the training subjects is derived using the feature extractor.

9. A method according to claim 8, wherein one of:

a) the machine learning algorithm is a regression algorithm, and the machine-learning algorithm determines the sleep state of the test subject using a continuous measure of sleep depth, optionally wherein the regression algorithm is a neural network;

b) the training data in respect of each training subject are temporally divided into a plurality of epochs, each epoch being labelled with one of a set of sleep states, optionally wherein the set of sleep states comprises wake, rapid-eye movement (REM) sleep, light non-rapid eye movement (NREM) sleep, or deep NREM sleep, further optionally wherein the set of sleep states further comprises intermediate NREM sleep; and the machine learning algorithm is a classification algorithm that classifies the sleep state of the test subject as one of the set of sleep states, optionally wherein the classification algorithm is one of a logistic regression, k-nearest neighbours, a random forest classifier, and a neural network; and

c) the training data in respect of each training subject are temporally divided into a plurality of epochs, each epoch being labelled with one of a set of sleep states, optionally wherein the set of sleep states comprises wake, rapid-eye movement (REM) sleep, light non-rapid eye movement (NREM) sleep, or deep NREM sleep, further optionally wherein the set of sleep states further comprises intermediate NREM sleep; the machine learning algorithm is a classification algorithm, optionally one of a logistic regression, k-nearest neighbours, a random forest classifier, and a neural network; the classification algorithm generates scores for each sleep state of the set of sleep states, the scores representing a confidence of the sleep state of the test subject being that sleep state of the set of sleep states; and the machine-learning algorithm determines the sleep state of the test subject using a continuous measure of sleep depth, the continuous measure being determined from a weighted combination of the scores for each sleep state of the set of sleep states.

10. A method according to claim 8 or 9, wherein the at least one cardiorespiratory feature for each of the training subjects is derived from training cardiorespiratory signals using the feature extractor, optionally wherein the training cardiorespiratory signals comprise one or more of heart rate, respiratory rate, a pulse waveform, and a respiratory waveform,

optionally wherein one or both of:

a) the training cardiorespiratory signals comprise a first plurality of training cardiorespiratory signals and a second plurality of training cardiorespiratory signals, wherein the first and second pluralities of training cardiorespiratory signals differ in one or more signal characteristics, optionally wherein the signal characteristics comprise sampling rate and duration; and

b) the training cardiorespiratory signals are derived from video images of the training subjects.

11. A method according to any one of claims 8 to 10, further comprising training the machine-learning algorithm using the training data.

12. A method of training a machine-learning algorithm to determine a sleep state of a subject, wherein:

the method uses training data in respect of a plurality of training subjects;

the training data are derived from video images of the training subjects;

the training data comprise for each of the training subjects: at least one measure of subject movement; and at least one cardiorespiratory feature of the subject;

the at least one cardiorespiratory feature for each of the training subjects is derived using a feature extractor trained using reference cardiorespiratory signals for each of a plurality of reference subjects, the feature extractor comprising a neural network, and the reference cardiorespiratory signals derived from time-resolved measurements from a plurality of sensors worn by the reference subjects; and

the method comprises training the machine learning algorithm using the training data.

13. A method according to claim 11 or 12, further comprising one or both of:

a) deriving the at least one cardiorespiratory feature for each of the training subjects from training cardiorespiratory signals using the feature extractor, optionally wherein the training cardiorespiratory signals comprise one or more of heart rate, respiratory rate, a pulse waveform, and a respiratory waveform; and
b) deriving the training cardiorespiratory signals and/or the at least one measure of subject movement from the video images of the training subjects.

14. A method according to any one of the preceding claims, wherein the at least one measure of test subject movement comprises one or more of a measure of subject movement in a head region of the subject, a measure of movement in a torso region of the subject, and a measure of movement in an outer region of the video image.

15. A computer apparatus configured to carry out a method according to any one of the preceding claims, or a computer program comprising, or a computer-readable storage medium having stored thereon, instructions which, when carried out by a computer, cause the computer to carry out a method according to any one of the preceding claims.

Fig. 1

Fig. 2

Fig. 3

Fig. 4

Fig. 5

a)   b)

Fig. 6

a)

b)

Fig. 7

Fig. 8

2 — Video images

S2 — Derive CR signals

Derive measures of movement — S4

5 — Input CR signals

S6 — Derive cardiorespiratory (CR) features

8 — CR feature

Measures of movement — 6

40 — Input data

Trained ML algorithm — 50

Determine sleep state — S30

Sleep state — 60

Fig. 9

Fig. 10

Fig. 11

Fig. 12

Fig. 13

Fig. 14

Fig. 15

**Fig. 16**

**Fig. 17**

Fig. 18(a)

Fig. 18(b)

Acc=91.06%

Fig. 19

Acc=83.11%

Fig. 20

Fig. 21

Fig. 22

Fig. 23

Fig. 24

Fig. 25

Fig. 26

Fig. 27

Fig. 28

Fig. 29

Fig. 30

Europäisches Patentamt

European Patent Office

Office européen des brevets

# EUROPEAN SEARCH REPORT

Application Number

EP 24 16 0886

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/278319 A1 (JACKSON LAB [US]; UNIV PENNSYLVANIA [US]) 5 January 2023 (2023-01-05) * page 8, paragraph 3 * * page 34 - page 35 * ----- | 1-15 | INV. A61B5/00 A61B5/024 A61B5/08 A61B5/11 A61B5/113 |
| X | NOCHINO TERUAKI ET AL: "Sleep stage estimation method using a camera for home use", BIOMEDICAL ENGINEERING LETTERS, THE KOREAN SOCIETY OF MEDICAL AND BIOLOGICAL ENGINEERING, KOREA, vol. 9, no. 2, 24 April 2019 (2019-04-24), pages 257-265, XP036784118, ISSN: 2093-9868, DOI: 10.1007/S13534-019-00108-W [retrieved on 2019-04-24] * abstract * ----- | 1-15 | |
| X,P | CARTER JONATHAN ET AL: "Deep Learning-Enabled Sleep Staging From Vital Signs and Activity Measured Using a Near-Infrared Video Camera", 2023 IEEE/CVF CONFERENCE ON COMPUTER VISION AND PATTERN RECOGNITION WORKSHOPS (CVPRW), IEEE, 17 June 2023 (2023-06-17), pages 5940-5949, XP034397599, DOI: 10.1109/CVPRW59228.2023.00632 [retrieved on 2023-08-15] * the whole document * ----- | 1-15 | **TECHNICAL FIELDS SEARCHED (IPC)** A61B |

The present search report has been drawn up for all claims

| Place of search | Date of completion of the search | Examiner |
|---|---|---|
| Berlin | 15 July 2024 | Clevorn, Jens |

EPO FORM 1503 03.82 (P04C01)

**ANNEX TO THE EUROPEAN SEARCH REPORT
ON EUROPEAN PATENT APPLICATION NO.**

EP 24 16 0886

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2024

| Patent document cited in search report | | Publication date | Patent family member(s) | | Publication date |
|---|---|---|---|---|---|
| WO 2023278319 A1 | | 05-01-2023 | AU 2022301046 A1 | | 18-01-2024 |
| | | | CA 3224154 A1 | | 05-01-2023 |
| | | | CN 117545417 A | | 09-02-2024 |
| | | | EP 4340711 A1 | | 27-03-2024 |
| | | | KR 20240027726 A | | 04-03-2024 |
| | | | WO 2023278319 A1 | | 05-01-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 19220087

**Non-patent literature cited in the description**

- **YOSINSKI J ; CLUNE J ; BENGIO Y ; LIPSON H.** How transferable are features in deep neural networks?. *arXiv: 1411.1792,* 06 November 2014
- **HE K ; ZHANG X ; REN S ; SUN J.** Deep Residual Learning for Image Recognition. *Proceedings of the IEEE Conference on Computer Vision and Pattern Recognition.,* 2016, 770-8
- **JORGE, J. ; VILLARROEL, M. ; TOMLINSON, H. et al.** Non-contact physiological monitoring of post-operative patients in the intensive care unit. *Digit. Med.,* 2022, vol. 5, 4, https://doi.org/10.1038/s41746-021-00543-z
- **VILLARROEL, M. ; JORGE, J. ; MEREDITH, D. et al.** Non-contact vital-sign monitoring of patients undergoing haemodialysis treatment. *Sci Rep,* 2020, vol. 10, 18529, https://doi.org/10.1038/s41598-020-75152-z
- **W. ZONG ; T. HELDT ; G. B. MOODY ; R. G. MARK.** An open-source algorithm to detect onset of arterial blood pressure pulses. *Computers in Cardiology,* 2003, vol. 2003, 259-262
- **RADHA M ; FONSECA P ; MOREAU A ; ROSS M ; CERNY A ; ANDERER P ; LONG X ; AARTS R M.** A deep transfer learning approach for wearable sleep stage classification with photoplethysmography. *Digital Medicine,* 15 September 2021, vol. 4 (1
- **SRIDHARN ; SHOEB A ; STEPHENS P ; KHARBOUCH A ; SHIMOL D B ; BURKART J ; GHOREYSHI A ; MYERS L.** Deep learning for automated sleep staging using instantaneous heart rate. *Digital Medicine.,* 20 August 2020, vol. 3 (1
- **NOCHINO T ; OHNO Y ; KATO T ; TANIIKE M ; OKADA S.** Sleep stage estimation method using camera for home use. *Biomedical Engineering Letters,* 24 April 2019, vol. 9, 257-65
- **XIAOLI CHEN ; RUI WANG ; PHYLLIS ZEE ; PAMELA L. LUTSEY ; SOGOL JAVAHERI ; CARMELA ALCÁNTARA ; CHANDRA L. JACKSON ; MICHELLE A. WILLIAMS ; SUSAN REDLINE.** Racial/Ethnic Differences in Sleep Disturbances: The Multi-Ethnic Study of Atherosclerosis (MESA). *Sleep,* 2015, vol. 38 (6), 877-888
- **KEVIN KOTZEN ; PETER H. CHARLTON ; SHARON SALABI ; LEA AMAR ; AMIR LANDESBERG ; JOACHIM A. BEHAR.** Sleep PPG-Net: A Deep Learning Algorithm for Robust Sleep Staging From Continuous Photoplethysmography. *IEEE Journal of Biomedical and Health Informatics,* 2023, vol. 27 (2), 924-932
- **JESSIE P. BAKKER ; MARCO ROSS ; RAY VASKO ; ANDREAS CERNY ; PEDRO FONSECA ; JEFF JASKO ; EDMUND SHAW ; DAVID P. WHITE ; PETER ANDERER.** Estimating sleep stages using cardiorespiratory signals: validation of a novel algorithm across a wide range of sleep-disordered breathing severity. *Journal of Clinical Sleep Medicine,* 2021, vol. 17 (7), 1343-1354
- **YUQI NIE ; NAM H.NGUYEN ; PHANWADEE SINTHONG ; JAYANT KALAGNANAM.** *A Time Series is Worth 64Words: Long-term Forecasting with Transformers,* 2022